# EUROPÄISCHE PATENTSCHRIFT

(11) **EP 2 004 136 B1**
(45) Veröffentlichungstag und Bekanntmachung des Hinweises auf die Patenterteilung: **30.04.2014**
(21) Anmeldenummer: 07723320.3
(22) Anmeldetag: 16.03.2007
(51) Int. Cl.: A61K 8/35, A61K 8/49, A61K 8/60, A61K 8/37, A61Q 19/04, A61Q 13/00

(54) **Verwendung von Flavonoiden**
Use of flavonoids
Utilisation de flavonoïdes

(30) Priorität: 13.04.2006 DE 102006017879
(43) Veröffentlichungstag der Anmeldung: 24.12.2008
(73) Patentinhaber: Merck Patent GmbH, 64293 Darmstadt (DE)
(72) Erfinder: RUDOLPH, Thomas, 64291 Darmstadt (DE); BUCHHOLZ, Herwig, 60599 Frankfurt (DE)
(86) Internationale Anmeldenummer: PCT/EP2007/002340
(87) Internationale Veröffentlichungsnummer: WO 2007/118565

(56) Entgegenhaltungen:
- WO-A-95/22960
- WO-A-95/26178
- WO-A-02/096371
- DE-A- 19 739 349
- DE-A- 19 755 504
- DE-A1-102004 002 170
- FR-A- 2 834 639
- US-A1- 2005 089 484
- DATABASE EPODOC EUROPEAN PATENT OFFICE, THE HAGUE, NL; XP002439064 & JP 04 363395 A (HASEGAWA T. CO. LTD. ET AL.) 16. Dezember 1992 (1992-12-16)
- DATABASE WPI Week 198524 Derwent Publications Ltd., London, GB; AN 1985-143934 XP002439068 & JP 60 078553 A (ASAMA KASEI KK) 4. Mai 1985 (1985-05-04)
- DATABASE WPI Week 200359 Derwent Publications Ltd., London, GB; AN 2003-621181 XP002439069 & JP 2003 104899 A (SANKI SHOJI KK) 9. April 2003 (2003-04-09)
- DATABASE EPODOC EUROPEAN PATENT OFFICE, THE HAGUE, NL; XP002439065 & JP 07 165547 A (LION CORP.) 27. Juni 1995 (1995-06-27)

## Beschreibung

Die vorliegende Erfindung betrifft die Verwendung von mindestens einem Flavonoid zur Geruchsverbesserung oder Geruchsstabilisierung, von Zubereitungen oder Vormischungen von Zubereitungen, enthaltend mindestens eine Selbstbräunungssubstanz, nach Anspruch 1.

Riechstoffe, Aromastoffe, allgemein Parfümöle, Duftstoffe oder Aroma Chemicals, sind aus dem menschlichen Alltag nicht mehr wegzudenken. Sie spielen in der menschlichen Kultur seit jeher eine wichtige Rolle, zuerst in kultischen Gebräuchen, wenig später auch in der Schönheitspflege. Auch in unserer Zeit besitzen sie eine herausragende und immer weiter zunehmende Bedeutung z. B. im Bereich der Produktbeduftung, im Bereich der Körperpflegeprodukte, insbesondere auch als eigentliche "Parfüms" oder im Bereich der Wasch- und Reinigungsmittel.

Entscheidend für die Wahrnehmbarkeit eines Riechstoffs ist seine Flüchtigkeit, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms während des Verdampfens, wobei man die Geruchseindrücke in "Kopfnote" ("top note"), "Herz¬ bzw. Mittelnote" ("middle note" bzw. "body") sowie "Basisnote" ("end note" bzw. "dry out") unterteilt. Da die Geruchswahmehmung, die bei Menschen sehr individuell ist, zu einem großen Teil auch auf der Geruchsintensität beruht, wird die Kopfnote eines Parfüms naturgemäß von leichtflüchtigen Verbindungen bestimmt, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird.

Chemisch handelt es sich bei den Parfümölen oft um reaktionsfähige Verbindungen vom Typ der Ester, Ether, Aldehyde, Ketone, Alkohole und Kohlenwasserstoffe. Gerade bei Lagerung, oder in Gegenwart weiterer Verbindungen - Insbesondere im Umfeld der Produktbeduftung - können Duftstoffe reagieren bzw. durch Reaktion anderer Inhaltsstoffe Nebengerüche entstehen.

Aroma Chemicals umfassen Duftstoffe und Geschmackstoffe.

Die Aufgabe der vorliegenden Erfindung war dementsprechend, Möglichkeiten zu finden, den Duft oder Geruch von Zubereitungen oder Vormischungen zu stabilisieren oder zu verbessern.

Jetzt wurde überraschend gefunden, dass dies durch die Verwendung von mindestens einem Flavonoid mit einem ungeladenen Flavangrundkörper gelingt, wie in Anspruch 1 beschrieben.

Ein erster Gegenstand der vorliegenden Erfindung ist daher die Verwendung von mindestens einem Flavonoid mit einem ungeladenen Flavangrundkörper zur Geruchsverbesserung und/oder Geruchsstabilisierung von Zubereitungen oder Vormischungen von Zubereitungen nach Anspruch 1.

Das Flavonoid wirkt dabei als Stabilisator für Selbstsbräunungssubstanzen, und/oder reduziert oder vermeidet lagerabhängige Fehlgerüche von Weiterhin kann das Flavonoid unangenehme Eigengrüche von Wirkstoffen, insbesondere von kosmetischen Rohstoffen, maskieren.

Es ist erfindungsgemäß bevorzugt, wenn die Geruchsverbesserung bei kosmetischen, dermatologischen oder pharmazeutischen Zubereitungen oder bei Vormischungen für kosmetische, dermatologische oder pharmazeutische Zubereitungen stattfindet.

Die Verwendung von Flavonoiden insbesondere in der Kosmetik bzw. Pharmazie ist an sich bekannt. So beschreibt beispielsweise die DE-A-1 9739349 die Verwendung von Troxerutin als Antioxidans oder Radikalfänger in kosmetischen und dermatologischen Zubereitungen.

DE 10 2004 002170 A1 beschreibt die Verminderung des unangenehmen Geruches von Dihydroxyaceton in Selbstbräunungszubereitungen durch einen Zusatz von Octylsalicylat.

Der Begriff "Flavonoid" im Sinne dieser Erfindung umfasst insbesondere Verbindungen, die sich aufgrund ihrer Grundstruktur zu folgenden Gruppen zuordnen lassen:
- Flavanone
- Flavan-3-ole (Catechine)
- Flavone
- Flavan-3,4-diole (Leukoanthocyanidine)
- Flavonole (3-Hydroxy-flaven-4-on)
- Flavanonale

Der Name "Flavonoid" leitet sich vom lateinischen Wort flavus= gelb ab und berücksichtigt damit die Tatsache, dass die meisten dieser Substanzen in ihrer reinen Form eine gelbliche Farbe aufweisen.

Beispielhaft sollen folgende Flavonoide angeführt werden: 5-Hydroxy-7,4'-dimethoxyflavon-8-sulfat, 7,8-dihydroxyflavon, Luteolin (Flavone); Catechin, Epicatechin, EpiGalloCatechinGallat (EGCG, TEAVIGO^{®} DSM) (Flavan-3-ole bzw. Flavan-3-ol-derivate); Kämpferol (Flavonol); Taxifolin (Flavanonol), sowie Naringenin (Flavanon), und Glycoside von Naringenin, beispielsweise Naringin-7-neohesperidosid.

Bevorzugte Flavonoide leiten sich von folgenden Gruppen ab:
- Flavonole
- Flavonol-0-glycoside
- Flavonol-0-glycosid-enthaltende Extrakte

Flavonoide kommen meist als lösliche Glykoside im Zellsaft der Pflanze vor. Die bevorzugten Flavonoide umfassen auch Aglyka (zuckerfreie Strukturen) und Aglyka-Konjugate. Mögliche Aglyka-Konjugate sind Hydroxyl-Derivate, wobei die Hydroxylgruppen ganz oder teilweise alkyliert, methyliert, glycyliert, sulfatiert oder verestert sind. Neben Hydroxylderivaten kommen auch C-Derivate als Aglyka-Konjugate in Frage.

Besonders bevorzugt ist für die Gruppe der Flavonole das Aglykon Quercetin. Bei der Gruppe der Flavonol-0-glycoside sind die Flavonol-3-glycoside wie Rutin, α-Glucosylrutin, Tilirosid, Isoquercetin, Rutinsulfat, Trishydroxyethylrutin (Troxerutin) sowie deren Sulfate und Phosphate besonders bevorzugt.

Auch Flavonol-7- und -8-glycoside können verwendet werden.

Der Begriff "Rutinsuffat" umfasst Mono-, Di-, Tri-, Tetra- oder Polysulfate des Rutins bzw. Gemische dieser Rutinsulfate. Der Begriff "Troxerutin" umfasst Mono-, Di-, Tri- Tetra- oder Polyethoxylate des Rutins bzw. Gemische dieser Rutinethoxylate.

Erfindungsgemäß bevorzugt verwendet werden die Flavonoide, ausgewählt aus der Gruppe Quercetin, Rutin, Rutinsulfat, α-Glucosylrutin, Tilirosid, Troxerutin und/oder Isoquercetin.

Für die Gruppe der Flavonol- oder Flavonot-0-glycosid-enthaltenden Extrakte sind die Wirkstoffkombinationen Emblica, Licorice und/oder Rosskastanienextrakt bevorzugt. Emblica wird aus den Früchten des Laubbaumes Phyllanthus emblica (auch Emblica officinalis) z.B. in Indien, China, Pakistan oder Nepal gewonnen. Die Hauptinhaltsstoffe von Emblica sind die niedermolekularen Gerbsäuren Emblicanin A und B, die das in der Haut vorkommende Eisen in Form von Komplexen binden. Bevorzugte Emblica- Lösungen sind kommerziell z.B. als EMBLICA^{®} (MERCK) oder CAPROS^{®} (siehe z.B, US-6,235,721 oder US-6,124,268) erhältlich. Prinzipiell kommen alle Emblica-Mischungen allein oder in Kokmbination mit mindestens einem Flavonoid für eine erfindungsgemäße Verwendung In Frage.

Das Licorice-Extrakt enthält das Flavonoid Glabridin (ein Stearyl Glycyrhetinat) und/oder Licochalcon A.

Das Rosskastanienextrakt enthält z.B. Esculin sowie weitere Flavonol- und/oder Flavonolglycosidbestandteile.

Solche pflanzlichen Substanzgemische können in dem Fachmann geläufiger Weise beispielsweise durch Auspressen oder Extrahieren der entsprechenden Pflanzen gewonnen werden.

Bevorzugt wird das mindestens eine Flavonoid für die erfindungsgemäße Verwendung in entsprechenden Formulierungen in einer Gesamtmenge von 0.01 bis 10 Gew.-%, noch bevorzugter in einer Menge von 0.1 bis 5 Gew.-% eingesetzt. Bevorzugt wird das mindestens eine Flavonoid für die erfindungsgemäße Verwendung in Vormischungen für entsprechende Formulierungen in einer Gesamtmenge von 1 bis 95% Gew.-%, bevorzugt in Mengen von 10 bis 50 Gew.- % oder 1 bis 10 Gew.-%, besonders bevorzugt in Mengen von 15 bis 33 Gew.-% oder 1,5 bis 5 Gew.-% eingesetzt. Welcher bevorzugte Parameterbereich in der Vormischung eingsetzt wird hängt von der Verwendung der Vormischung bzw. der Verwendung der Formulierung enthaltend die Vormischung ab. Die entsprechende Auswahl kann von dem entsprechenden Fachmann unter Zuhilfenahme seines Fachwissens erfolgen.

Im Sinne der vorliegenden Erfindung wird neben dem Begriff Parfümöl gleichbedeutend auch der Begriff Duftstoff verwendet.

Unter einer Vormischung für eine Zubereitung oder Formulierung wird eine Feststoffabmischung von beispielsweise pulverförmigen Inhaltsstoffen verstanden, die als Pulvermischung als solche mit mindestens einem geeigneten Träger und gegebenenfalls weiteren Inhaltsstoffen gemischt wird und so eine Zubereitung hergestellt wird.

Erfindungsgemäß kann jedoch das mindestens eine Flavonoid auch in Vormischungen eingesetzt werden, deren Aggregatzustand bei Raumtemperatur nicht fest oder pulverförmig sind, beispielsweise enthaltend eine flüssige Komponente, deren Vormischung mit anderen Komponenten jedoch selbst wieder sowohl fest als auch flüssig sein kann.

Unter einer Zubereitung wird eine Mischung aus dem erfindungsgemäß verwendeten mindestens einen Flavonoid zusammen mit der Komponente, deren Geruch verbessert und/oder stabilisiert werden soll und mindestens einem für kosmetische, dermatologische oder pharmazeutische bekannten Träger verstanden.

Dabei kann es erfindungsgemäß bevorzugt sein, wenn es sich bei dem mindestens einen Flavonoid um ein Flavonoid handelt, bei dem eine oder mehrere phenolische Hydroxygruppen durch Veretherung oder Veresterung blockiert sind. Beispielsweise Hydroxyethyl-substituierte Flavonoide, wie vorzugsweise Troxerutin, Troxequercetin, Troxeisoquercetin oder Troxeluteolin, und Flavonoidsulfate oder Flavonoid-phosphate, wie vorzugsweise Rutinsulfate, haben sich dabei als besonderes gut geeignete Flavonoide im Sinne der vorliegenden Erfindung erwiesen.

Besonders bevorzugt im Sinne der erfindungsgemäßen Verwendung sind Rutinsulfat und Troxerutin. Ganz besonders bevorzugt ist die Verwendung von Troxerutin.

Weiter verfügen erfindungsgemäß bevorzugte Flavonoide über einen nicht positiv geladenen Flavangrundkörper. Es wird vermutet, dass durch diese Flavonoide Metallionen wie z.B. Fe²⁺/Cu²⁺ komplexiert werden und so Autooxidationsvorgänge bei Verbindungen, deren Abbau zu Fehlgerüchen führt, verhindert bzw. vermindert werden.

Weitere bevorzugte Kombinationen von Ausführungsformen sind in den Ansprüchen offenbart.

Erfindungsgemäß besonders bevorzugt ist es dabei, wenn die Zubereitung mindestens einen Wirkstoff vom Typ der Aldehyde oder Ketone enthält, da festgestellt wurde dass Flavonoide solche Verbindungen in besonderer Weise stabilisieren können. Besonders signifikant ist die Geruchsverbesserung einer Zubereitung enthaltend Dihydroxyaceton oder Vanillin.

Die Geruchsverbessetung findet erfindungsgemäß in Zubereitungen oder Vormischungen für Zubereitungen statt, die mindestens in Flavonoid mit einem ungeladenem Flavangrundkörper und mindestens eine Selbstbräunungssubstanz enthalten, die durch die Anwesenheit des Flavonoids stabilisert wird. Entsprechende abbaubare Stoffe bzw. deren Abbauprodukte führen oft zu Fehlgerüchen in Zubereitungen. Bevorzugte Selbstbräunungssubstanzen weisen dabei wiederum Aldehyd- oder Ketonfunktionen auf.

Als Selbstbräunungssubstanzen können unter anderem eingesetzt werden:

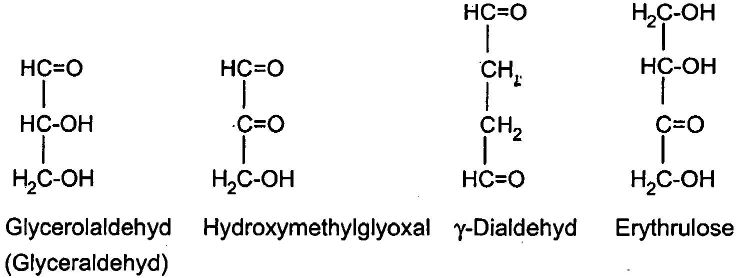

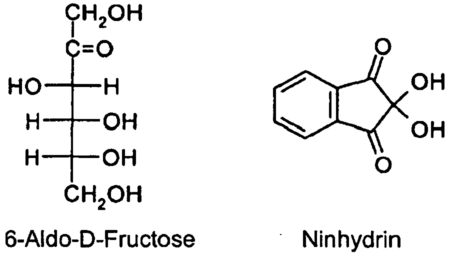

Ferner ist das 5-Hydroxy-1,4-naphtochinon (Juglon) zu nennen, das aus den Schalen frischer Walnüsse extrahiert wird, 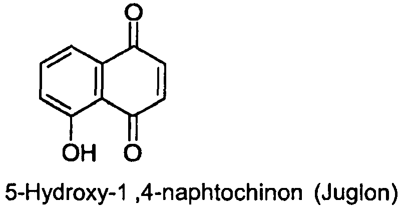 sowie das in den Henna-Blättern vorkommende 2-Hydroxy-1,4-naphtochinon (Lawson).

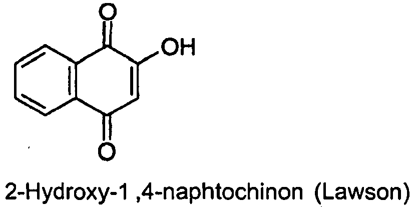

Bevorzugt werden folgende Triosen und Tetrosen eingesetzt: 1,3-Diyhydroxyaceton (DHA oder auch Dihydroxyaceton) und dessen Derivate, Glyceraldehyd, Dihydroxyacetonphosphat, Glyceraldehydphosphat, Erythrose und 1,3,4-Trihydroxy-2-butanon (Erythrulose). Ganz besonders bevorzugt in Vormischungen oder Zubereitungen stabilisiert werden Erythrulose und/oder 1,3-Dihydroxyaceton (DHA). DHA ist ein im menschlichen Körper vorkommender dreiwertiger Ketozucker.

Dabei kann es im Sinne der vorliegenden Erfindung insbesondere auch bevorzugt sein, wenn Vormischungen für Zubereitungen enthaltend erfindungsgemäß mindestens ein Flavonoid nach Anspruch 1 und eine oder mehrere der hier beschriebenen Selbstsbräunungssubstanzen eingesetzt werden. In solchen Vormischungen beträgt das Verhältnis Selbstsbräunungssubstanz zu Flavonoid vorzugsweise 5 : 1 bis 1 : 2. Entsprechende Vormischungen sind in der Deutschen Patentanmeldung mit dem Anmeldeaktenzeichen DE 102005035683.4, sowie den U.S. Patentanmeldungen mit den Aktenzeichen 60/702,983 und 60/748,588 beschrieben, deren diesbezüglicher Inhalt ausdrücklich auch zum Offenbarungsgehalt der vorliegenden Anmeldung gehört.

Eine weitere entsprechende Vormischung ist beispielsweise auch eine Feststoffabmischung enthaltend mindestens eine Selbstbräunungssubstanz, beispielsweise DHA und/oder Erythrulose, einen Formaldehydfänger und erfindungsgemäß das Flavonoid. Geeignete Formaldehydfänger sind beispielsweise D-Glucitol, Glycerol, 1,2-Propandiol, Isopropylalkohol, Ethylenglycol, Diethylenglycol, Resorcinol, Pyrogallol, Phloroglucin, Hydrogensulfite, Sulfite, Mischungen von Sulfiten und Sulfiden, Bisulfite, Transresveratrol, Harnstoff, Benzotriazole oder Weizengluten, insbesondere Bisulfite. Eine bekannte Feststoffabmischung in diesem Sinne ist DHA Plus, eine Selbstbräunungsvormischung vertrieben durch Merck, Darmstadt, welche aus Dihydroxyaceton, Natriumbisulfit und Magnesiumstearat besteht, welcher erfindungsgemäß ein Flavonoid nach Anspruch 1 zur Geruchsverbesserung zugegeben werden kann.

Erfindungsgemäß kann ein Flavonoid nach Anspruch 1 auch zu Vormischungen oder Zubereitungen zugegeben werden, die mindestens eine Selbstbräunungssubstanz und Dimethylisosorbid enthalten.

In den beschriebenen Vormischungen, die erfindungsgemäß zusätzlich mindestens ein Flavonoid nach Anspruch 1 enthalten, können weiterhin auch Pigmente enthalten sein, wobei der Schichtaufbau der Pigmente nicht limitiert ist.

Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramerinblau und/oder Manganviolett. Es ist insbesondere vorteilhaft, die Farbstoffe und/oder Farb¬ pigmente aus der folgenden Liste zu wählen. Die Colour Index Nummern (CIN) sind dem Rowe Colour Index, 3. Auflage, Society of Dyers and Colourists, Bradford, England, 1971 entnommen.

| **Chemische oder sonstige Bezeichnung** | **CIN** | **Farbe** |
|---|---|---|
| Pigment Green | 10006 | grün |
| Acid Green 1 | 10020 | Grün |
| 2,4-Dinitrohydroxynaphthalin-7-sulfonsäure | 10316 | Gelb |
| Pigment Yellow 1 | 11680 | Gelb |
| Pigment Yellow 3 | 11710 | Gelb |
| Pigment Orange 1 | 11725 | Orange |
| 2,4-Dihydroxyazobenzol | 11920 | Orange |
| Solvent Red 3 | 12010 | Rot |
| 1-(2'-Chlor-4'-nitro-1'-phenylazo)-2-hydroxynaphthalin | 12085 | Rot |
| Pigment Red 3 | 12120 | Rot |
| Ceresrot; Sudanrot; Fettrot G | 12150 | Rot |
| Pigment Red 112 | 12370 | Rot |
| Pigment Red 7 | 12420 | Rot |
| Pigment Brown 1 | 12480 | Braun |
| 4-(2'-Methoxy-5'sulfonsäurediethylamid-1'-phenylazo)-3-hydroxy-5"-chloro-2",4"-dimethoxy2-naphthoesäureanilid | 12490 | Rot |
| Disperse Yellow 16 | 12700 | Gelb |
| 1-(4-Sulfo-1-phenylazo)-4-amino-benzol-5-sulfosäure | 13015 | Gelb |
| 2,4-Dihydroxy-azobenzol-4'-sulfosäure | 14270 | Orange |
| 2-(2,4-Dimethylphenylazo-5-sulfosaure)-1-hydroxynaphthalin-4-sulfosäure | 14700 | Rot |
| 2-(4-Sulfo-1-naphthylazo)-1-naphthol-4-sulfosäure | 14720 | Rot |
| 2-(6-Sulfo-2,4-xylylazo)-1-naphthol-5-sulfosäure | 14815 | Rot |
| 1-(4'-Sulfophenylazo)-2-hydroxynaphthalin | 15510 | Orange |
| 1-(2-Sulfosäure-4-chlor-5-carbonsäure-1-phenylazo)-2-hydroxynaphthalin | 15525 | Rot |
| 1-(3-Methyl-phenylazo-4-sulfosäure)-2-hydroxynaphthalin | 15580 | Rot |
| 1-(4',(8')-Sulfosaurenaphthylazo)-2-hydroxynaphthalin | 15620 | Rot |
| 2-Hydroxy-1,2'-azonaphthalin-1'-sulfosäure | 15630 | Rot |
| 3-Hydroxy-4-phenylazo-2-naphthylcarbonsäure | 15800 | Rot |
| 1-(2-Sulfo-4-methyl-1-phenylazo)-2-naphthylcarbonsaure | 15850 | rot |
| 1-(2-Sulfo-4-methyl-5-chlor-1-phenylazo)-2-hydroxy-naphthalin-3-carbonsäure | 15865 | Rot |
| 1-(2-Sulfo-1-naphthylazo)-2-hydroxynaphthalin-3-carbonsäure | 15880 | Rot |
| 1-(3-Sulfo-1-phenylazo)-2-naphthol-6-sulfosäure | 15980 | Orange |
| 1-(4-Sulfo-1-phenylazo)-2-naphthol-6-sulfosäure | 15985 | Gelb |
| Allura Red | 16035 | Rot |
| 1-(4-Sulfo-1-naphthylazo)-2-naphthol-3,6-disulfosäure | 16185 | Rot |
| Acid Orange 10 | 16230 | Orange |
| 1-(4-Sulfo-1-naphthylazo)-2-naphthol-6,8-disulfosäure | 16255 | Rot |
| 1-(4-Sulfo-1-naphthylazo)-2-naphthol-3,6,8-trisulfosäure | 16290 | Rot |
| 8-Amino-2-phenylazo-1-naphthol-3,6-disulfosäure | 17200 | Rot |
| Acid Red 1 | 18050 | Rot |
| Acid Red 155 | 18130 | Rot |
| Acid Yellow 121 | 18690 | Gelb |
| Acid Red 180 | 18736 | Rot |
| Acid Yellow 11 | 18820 | Gelb |
| Acid Yellow 17 | 18965 | Gelb |
| 4-(4-Sulfo-1-phenylazo)-1-(4-sulfophenyl)-5-hydroxy-pyrazolon-3-carbonsäure | 19140 | Gelb |
| Pigment Yellow 16 | 20040 | Gelb |
| 2,6-(4'-Sulfo-2",4"-dimethyl)-bis-phenylazo)1,3-dihydroxybenzol | 20170 | Orange |
| Acid Black 1 | 20470 | Schwarz |
| Pigment Yellow 13 | 21100 | Gelb |
| Pigment Yellow 83 | 21108 | Gelb |
| Solvent Yellow | 21230 | Gelb |
| Acid Red 163 | 24790 | Rot |
| Acid Red 73 | 27290 | Rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4-[4"-Sulfo-1"-phenylazo)-7'-sulfo-1'-naphthylazo]-1-hydroxy-8-acetyl-aminonaphthalin-3,5-disulfosäure | 28440 | Schwarz |
| Direct Orange 34, 39, 44, 46, 60 | 40215 | Orange |
| Food Yellow | 40800 | Orange |
| trans-ß-Apo-8'-Carotinaldehyd (C₃₀) | 40820 | Orange |
| trans-Apo-8'-Carotinsäure (C₃₀)-ethylester | 40850 | Orange |
| Canthaxanthin | 40850 | Orange |
| Acid Blue 1 | 42045 | Blau |
| 2,4-Disulfo-5-hydroxy-4'-4"-bis-(diethylamino)triphenyl-carbinol | 42051 | Blau |
| 4-[(-4-N-Ethyl-p-sulfobenzylamino)-phenyl-(4-hydroxy-2-sulfophenyl)-(methyten)-1-(N-ethylN-p-sulfobenzyl)-2,5-cyclohexadienimin] | 42053 | Grün |
| Acid Blue 7 | 42080 | Blau |
| (N-Ethyl-p-sulfobenzyl-amino)-phenyl-(2-sulfophenyl)-methylen-(N-ethyl-N-p-sulfo-benzyl) Δ^{2,5}-cyclohexadienimin | 42090 | Blau |
| Acid Green 9 | 42100 | Grün |
| Diethyl-di-sulfobenzyl-di-4-amino-2-chlor-di-2-methylfuchsonimmonium | 42170 | Grün |
| Basic Violet 14 | 42510 | Violet |
| Basic Violet 2 | 42520 | Violet |
| 2'-Methyl-4'-(N-ethyl-N-m-sulfobenzyl)-amino-4"-(N-diethyl)-amino-2-methyl-N-ethyllN-m-sulfobenzyl-fuchsonimmonium | 42735 | Blau |
| 4'-(N-Dimethyl)-amino-4"-(N-phenyl)-aminonaphtho-N-dimethylfuchsonimmonium | 44045 | Blau |
| 2-Hydroxy-3,6-disulfo-4,4'-bis-dimethylaminonaphthofuchsonimmonium | 44090 | Grün |
| Acid Red 52 | 45100 | Rot |
| 3-(2'-Methylphenylamino)-6-(2'-methyl-4'-sulfophenylamino)-9-(2"-carboxyphenyl)-xantheniumsalz | 45190 | Violet |
| Acid Red 50 | 45220 | Rot |
| Phenyl-2-oxyfluoron-2-carbonsäure | 45350 | gelb |
| 4,5-Dibromfluorescein | 45370 | Orange |
| 2,4,5,7-Tetrabromfluorescein | 45380 | Rot |
| Solvent Dye | 45396 | Orange |
| Acid Red 98 | 45405 | Rot |
| 3',4',5',6'-Tetrachlor-2,4,5,7-tetrabromfluorescein | 45410 | Rot |
| 4,5-Diiodfluorescein | 45425 | Rot |
| 2,4,5,7-Tetraiodfluorescein | 45430 | Rot |
| Chinophthalon | 47000 | Gelb |
| Chinophthalon-disulfosäure | 47005 | Gelb |
| Acid Violet 50 | 50325 | Violett |
| Acid Black 2 | 50420 | Schwarz |
| Pigment Violet 23 | 51319 | Violett |
| 1,2-Dioxyanthrachinon, Calcium-Sluminiumkomplex | 58000 | Rot |
| 3-Oxypyren-5,8,10-sulfosäure | 59040 | Grün |
| 1-Hydroxy-4-N-phenyl-aminoanthrachinon | 60724 | Violett |
| 1-Hydroxy-4-(4'-methylphenylamino)-anthrachinon | 60725 | Violett |
| Acid Violet 23 | 60730 | Violett |
| 1,4-Di(4'-methyl-phenylamino)-anthrachinon | 61565 | Grün |
| 1,4-Bis-(o-sulfo-p-toluidino)-anthrachinon | 61570 | Grün |
| Acid Blue 80 | 61585 | Blau |
| Acid Blue 62 | 62045 | Blau |
| N,N'-Dihydro-1,2,1',2'-anthrachinonazin | 69800 | Blau |
| Vat Blue 6; Pigment Blue 64 | 69825 | Blau |
| Vat Orange 7 | 71105 | orange |
| Indigo | 73000 | Blau |
| Indigo-disulfosäure | 73015 | Blau |
| 4,4'-Dimethyl-6,6'-dichlorthioindigo | 73360 | Rot |
| 5,5'Dichlor-7,7'-dimethylthioindigo | 73385 | violett |
| Quinacridone Violet 19 | 73900 | violett |
| Pigment Red 122 | 73915 | Rot |
| Pigment Blue 16 | 74100 | blau |
| Phthalocyanine | 74160 | blau |
| Direct Blue 86 | 74180 | blau |
| Chlonerte Phthalocyanine | 74260 | grün |
| Natural Yellow 6, 19; Natural Red 1 | 75100 | gelb |
| Bixin, Nor-Bixin | 75120 | orange |
| Lycopin | 75125 | gelb |
| trans-alpha-, bet- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsaure | 75470 | Rot |
| Chlorophyll,a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | Rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268 | schwarz |
| | :1 | |
| Chromoxid | 77288 | grün |
| Chromoxid, wasserhaltig | 77278 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyahoferrat | 77510 | blau |
| Pigment White 18 | 77713 | weiß |
| Mangananimoniumdiphosphat | 77742 | violett |
| Manganphosphat; Mn₃(PO₄)₂ 7 H₂O | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalzem, Anthocyane | | rot |
| Aluminium-, Zink-, Magnesium- und Calciumstearat | | weiß |
| Bromthymolblau | | blau |

Bevorzugt sind insbesondere die im folgenden aufgelisteten Arten von Perlglanzpigmenten:
1. Natürliche Perlglanzpigmente, wie z. B
   - "Fischsilber" (Guanin/Hypoxanthin-Mischkristalle aus Fischschuppen) und
   - "Perlmutt" (vermahlene Muschelschalen)
2. Monokristalline Perlglanzpigmente wie z. B. Bismuthoxychlorid (BiOCI)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| **Gruppe** | **Belegung/Schichtdicke** | **Farbe** |
|---|---|---|
| **Silberweiße Perlglanzpigmente** | TiO₂: 40-60 nm | silber |
| **Interferenzpigmente** | TiO₂: 60-80 nm | gelb |
| | TiO₂: 80-100 nm | rot |
| | TiO₂: 100-140 nm | blau |
| | TiO₂: 120-160 nm | grün |
| **Farbglanzpigmente** | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| **Kombinationspigmente** | TiO₂ / Fe₂O₃ | Goldtöne |
| | TiO₂ / Cr₂O₃ | grün |
| | TiO₂ / Berliner Blau | tiefblau |

Besonders bevorzugt sind z. B. die von der Firma Merck unter den Handelshamen Timiron®, Colorona®, Dichrona®, Xirona® oder Ronastar® erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Perlglanzpigmente, welche unter der Verwendung von Siθ₂ hergestellt werden. Solche Pigmente, die auch zusätzlich gonichromatische Effekte haben können, sind z. B. unter dem Handelsnamen Sicopearl Fantastico bei der Firma BASF erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks® erhältlich. Sie weisen durch ihre Partikelgröße von 40-80 µm zusätzlich zu der Farbe einen Glitzereffekt auf.

Besonders vorteilhaft sind ferner auch Effektpigmente, welche unter der Handelsbezeichnung Metasomes® Standard / Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Besonders geeignete Pigmente in den Vormischungen sind beispielsweise Ronastar® Silver oder Colorona® Bronze.

Selbstbräunerzubereitungen, insbesondere solche, die Dihydroxyaceton enthalten, neigen bei der Anwendung auf der menschlichen Haut zu - vermutlich durch Produkte von Nebenreaktionen verursachten - Fehlgerüchen, die von den Anwendern teilweise als unangenehm empfunden werden. Es hat sich gezeigt, dass diese Fehlgerüche bei Verwendung der erfindungsgemäß einzusetzenden Flavonoide vermieden werden. Daher ist die Verwendungen von mindestens einem Flavonoid nach Anspruch 1 zur Stabilisierung und/oder Verbesserung des Geruches von Selbstbräunerzubereitungen enthaltend mindestens eine Selbstbräunersubstanz auf der Haut ein weiterer Gegenstand der vorliegenden Erfindung.

Zu den bevorzugt einzusetzenden zusätzlichen Aktivsubstanzen zählen ferner z.B. UV-Filter, Chromon-Derivate, Aryloxime und Parabene.

Parabene sind 4-Hydroxybenzoesäureester, die in freier Form oder als NatriumSalze zur Konservierung von Zubereitungen im Bereich der Nahrungsmittel, Kosmetik und Arzneimittel verwendet werden. Die Wirkung der Ester ist direkt proportional zur Kettenlänge des Alkyl-Restes, umgekehrt nimmt jedoch die Löslichkeit mit steigender Kettenlänge ab. Als nicht dissoziierende Verbindungen sind die Ester weitgehend pH-Wert-unabhängig und wirken in einem pH-Bereich von 3,0-8,0. Der antimikrobielle Wirkmechanismus beruht auf einer Schädigung der Mikrobenmembranen durch die Oberflächenaktivität der PHB-Ester sowie auf der Eiweiß-Denaturierung. Daneben treten Interaktionen mit Coenzymen auf. Die Wirkung richtet sich gegen Pilze, Hefen und Bakterien. Die als Konservierungsmittel wichtigsten Parabene sind 4-Hydroxybenzoesäuremethylester, 4-Hydroxy-benzoesäureethylester, 4-Hydroxybenzoesäurepropylester, 4-Hydroxy-benzoesäurebutylester,

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE 41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psoriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die neben den genannten Verbindung(en) zusätzlich ein Aryloxim, vorzugsweise 2-Hydroxy-5-methyllaurophenonoxim, enthalten, zeigen überraschende antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew-% Aryloxim enthält.

Unter den Coumaranonen ist 4,6,3',4'-Tetrahydroxybenzylcoumaranon-3 bevorzugt.

Unter Chromon-Derivaten werden vorzugsweise bestimmte Chromen-2-on-Derivate, die sich als Wirkstoffe zur vorbeugenden Behandlung von menschlicher Haut und menschlicher Haare gegen Alterungsprozesse und schädigende Umwelteinflüssen eignen, verstanden. Sie zeigen gleichzeitig ein niedriges Irritationspotential für die Haut, beeinflussen die Wasserbindung in der Haut positiv, erhalten oder erhöhen die Elastizität der Haut und fördern somit eine Glättung der Haut. Diese Verbindungen entsprechen vorzugsweise der folgenden Formel 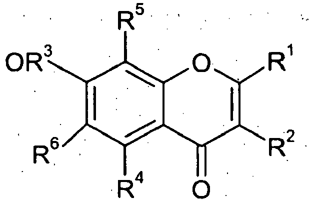 wobei
R¹ und R² gleich oder verschieden sein können und ausgewählt sind aus
   - H, -C(=O)-R⁷, -C(=O)-OR⁷,
   - geradkettigen oder verzweigten C₁- bis C₂₀-Alkylgruppen,
   - geradkettigen oder verzweigten C₃- bis C₂₀-Alkenylgruppen, geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
   - C₃- bis C₁₀-Cycloalkylgruppen und/oder C₃- bis C₁₂-Cycloalkenylgruppen, wobei die Ringe jeweils auch durch -(CH₂)ₙ-Gruppen mit n = 1 bis 3 überbrückt sein können,
R³ steht für H oder geradkettige oder verzweigte C₁- bis C₂₀-Alkylgruppen,
R⁴ steht für H oder OR⁸,
R⁵ und R⁶ gleich oder verschieden sein können und ausgewählt sind aus
   - -H, -OH,
   - geradkettigen oder verzweigten C₁- bis C₂₀-Alkylgruppen,
   - geradkettigen oder verzweigten C₃- bis C₂₀-Alkenylgruppen,
   - geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann und
R⁷ steht für H, geradkettige oder verzweigte C₁- bis C₂₀-Alkylgruppen, eine Polyhydroxy-Verbindung, wie vorzugsweise einen Ascorbinsäurerest oder glycosidische Reste und
R⁸ steht für H oder geradkettige oder verzweigte C₁- bis C₂₀-Alkylgruppen, wobei mindestens 2 der Substituenten R¹, R², R⁴-R⁶ verschieden von H sind oder mindestens ein Substituent aus R¹ und R² für -C(=O)-R⁷ oder -C(=O)-OR⁷ steht.

Der Anteil an einer oder mehreren Verbindungen ausgewählt aus Chromon-Derivaten und Coumaranonen in einer Zubereitung beträgt vorzugsweise von 0,001 bis 5 Gew.%, besonders bevorzugt von 0,01 bis 2 Gew.% bezogen auf die gesamte Zubereitung.

Alle Verbindungen oder Komponenten, die in den Zubereitungen verwendet werden können, sind entweder bekannt und käuflich erwerbbar oder können nach bekannten Verfahren synthetisiert werden.

Handelt es sich bei den Zubereitungen um topisch anwendbare Zubereitungen, beispielsweise kosmetische oder dermatologische Formulierungen, so enthalten die Zubereitungen in diesem Fall einen kosmetisch oder dermatologisch geeigneten Träger und je nach gewünschtem Eigenschaftsprofil optional weitere geeignete Inhaltsstoffe. Handelt es sich um pharmazeutische Zubereitungen, so enthalten die Zubereitung in diesem Fall einen pharmazeutisch verträglichen Träger und optional weitere pharmazeutische Wirkstoffe.

Die schützende Wirkung von Zubereitungen gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet geeignet schnell oder zeitverzögert wirkende Antioxidantien auszuwählen.

In einer bevorzugten Ausführungsform handelt es sich daher bei der Zubereitung um eine Zubereitung zum Schutz von Körperzellen gegen oxidativen Stress, insbesondere zur Verringerung der Hautalterung, dadurch gekennzeichnet, dass sie neben dem mindestens einen Flavonoid nach Anspruch 1 sowie der mindestens einen Selbstbräunungssubstanz und gegebenenfalls anderen Inhaltsstoffen ein oder mehrere Antioxidantien enthält.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Geeignete Antioxidantien sind auch Verbindungen der allgemeinen Formeln A oder B 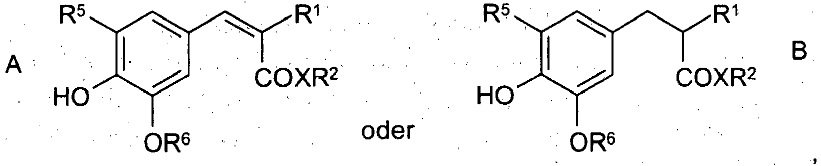 worin
R¹ aus der Gruppe -C(O)CH₃, -CO₂R³, -C(O)NH₂ und -C(O)N(R⁴)₂ ausgewählt werden kann,
X O oder NH,
R² lineares oder verzweigtes Alkyl mit 1 bis 30 C-Atomen,
R³ lineares oder verzweigtes Alkyl mit 1 bis 20 C-Atomen,
R⁴ jeweils ungabhängig voneinander H oder lineares oder verzweigtes Alkyl mit 1 bis 8 C-Atomen,
R⁵ lineares oder verzweigtes Alkyl mit 1 bis 8 C-Atomen oder lineares oder verzweigtes Alkoxy mit 1 bis 8 C-Atomen und
R⁶ lineares oder verzweigtes Alkyl mit 1 bis 8 C-Atomen bedeutet, vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex^{®} ST Liquid) und/oder 2-(4-Hydroxy-3,5-dimethoxybenzy)-malonsäure-bis-(2-ethylhexyl)ester (z.B. RonaCare^{®} AP).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex^{®} AP), natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004). Derartige Antioxidantien werden mit den erfindungsgemäßen Verbindungen in solchen Zusammensetzungen üblicherweise in Verhältnissen im Bereich von 1000:1 bis 1:1000, bevorzugt in Mengen von 100:1 bis 1:100 eingesetzt.

Unter den Phenolen, die verwendet werden können, sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R, Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH-Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers und I.M.C.M. Rietjens (Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete Antioxidantien sind ferner Verbindungen der Formel (III) 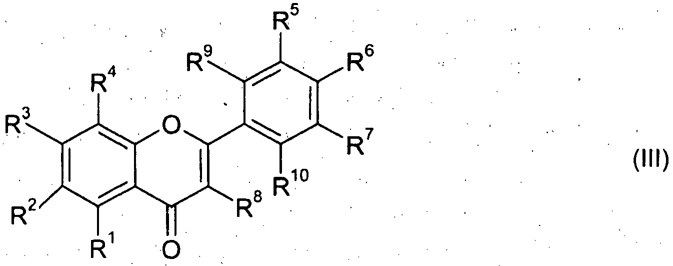 wobei R¹ bis R¹⁰ gleich oder verschieden sein können und ausgewählt sind aus
- H
- OR¹¹
- geradkettigen oder verzweigten C₁- bis C₂₀-Alkylgruppen,
- geradkettigen oder verzweigten C₃- bis C₂₀-Alkenylgruppen,
- geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- C₃- bis C₁₀-Cycloalkylgruppen und/oder C₃- bis C₁₂-Cycloalkenylgruppen, wobei die Ringe jeweils auch durch -(CH₂)ₙ-Gruppen mit n = 1 bis 3 überbrückt sein können,
- wobei alle OR¹¹ unabhängig voneinander stehen für
- OH
- geradkettige oder verzweigte C₁- bis C₂₀-Alkyloxygruppen,
- geradkettigen oder verzweigten C₃- bis C₂₀-Alkenyloxygruppen,
- geradkettigen oder verzweigten Cᵣ bis C₂o-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder
- C₃- bis Cio-Cycloalkyloxygruppen und/oder C₃- bis C₁₂-Cycloalkenyloxygruppen, wobei die Ringe jeweils auch durch -(CH₂)ₙ-Gruppen mit n = 1 bis 3 überbrückt sein können und/oder,
- Mono- und/oder Oligoglycosylreste,
   mit der Maßgabe, dass mindestens 4 Reste aus R¹ bis R⁷ stehen für OH und dass im Molekül mindestens 2 Paare benachbarter Gruppen -OH vorliegen,
- oder R², R⁵ und R⁶ für OH und die Reste R¹, R³, R⁴ und R⁷⁻¹⁰ für H stehen, wie sie in der Deutschen Patentanmeldung DE-A-102 44 282 beschrieben sind.

Die einzusetzenden Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B-i), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherol-hydrogensuccinat, Vitamin Ki, Esculin (Vitamin P-Wirkstoff), Thiamin (Vitamin B-i), Nicotinsäure (Niacin), Pyridoxin, Pyridoxal, Pyridoxamin, (Vitamin B₆), Panthothensäure, Biotin, Folsäure und Cobalamin (Vitamin B₁₂), insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den flavonoidhaltigen Vormischungen oder Zubereitungen üblicherweise bei kosmetischer Anwendung in Bereichen von 0,01 bis 5,0 Gew.-%, bezogen auf das Gesamtgewicht, zugesetzt.

Bevorzugte Zubereitungen können auch dem Sonnenschutz dienen und enthalten dann neben dem mindestens einen Flavonoid nach Anspruch 1 sowie der mindestens einen Selbstbräunungssubstanz und gegebenenfalls anderen Inhaltsstoffen auch UV-Filter.

Prinzipiell kommen alle UV-Filter für eine Kombination in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B. Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL),
Benzoyl- oder Dibenzoylmethane wie 1-(4-tert-Butylphenyl)-3-(4-methoxyphenyl)-propan-1,3-dion (z.B. Eusolex® 9020) oder 4-Isopropyldibenzoylmethan (z.B. Eusolex® 8020),
Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),
Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000),
Salicylatderivate wie 2-Ethylhexylsalicylat (z. B. Eusolex® OS), 4-Isopropylbenzylsalicylat (z.B. Megasol®) oder 3,3,5-Trimethylcyclohexylsalicylat (z.B. Eusolex® HMS),
4-Aminobenzoesäure und Derivate wie 4-Aminobenzoesäure, 4-(Dimethylamino)-benzoesäure-2-ethylhexylester (z.B. Eusolex® 6007), ethoxylierter 4-Amino-benzoesäureethylester (z.B. UVinul® P25),
Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;
und weitere Substanzen wie
- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin (z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden.

Weitere geeignete organische UV-Filter sind z.B.
- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®, Drometrizole, Trisiloxane, Mexoryl® XL),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb^{®} HEB),
- α-(Trimethylsilyl)-ω-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1)
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).
- 4,4-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb^{®} HEB),

Weitere geeignete UV-Filter sind auch Methoxyflavone ensprechend der Deutschen Patentanmeldung DE-A-10232595.

Organische UV-Filter werden in der Regel in einer Menge von 0,5 bis 20 Gewichtsprozent, vorzugsweise 1 - 15 %, in Formulierungen eingearbeitet.

Um einen optimierten UV-Schutz zu gewährleisten ist es weiter bevorzugt, wenn Zubereitungen mit Lichtschutzeigenschaften auch anorganische UV-Filter enthalten. Als anorganische UV-Filter sind solche aus der Gruppe der Titandioxide wie z.B. gecoatetes Titandioxid (z.B. Eusolex® T-2000, Eusolex^{®}T-AQUA, Eusolex® T-AVO), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 %, in kosmetische Zubereitungen eingearbeitet.

Bevorzugte Verbindungen mit UV-filternden Eigenschaften sind 3-(4'-Methylbenzyliden)-dl-kampfer, 1-(4-tert-Butylphenyl)-3-(4-methoxy-phenyl)-propan-1,3-dion, 4-Isopropyldibenzoylmethan, 2-Hydroxy-4-methoxy-benzophenon, Methoxyzimtsäureoctylester, 3,3,5-Trimethyl-cyclo-hexyl-sali-cylat, 4-(Dimethylamino)benzoesäure-2-ethyl-hexylester, 2-Cyano-3,3-di-phenyl-acrylsäure-2-ethylhexylester, 2-Phenyl-benzimidazol-5-sulfon-säure sowie ihre Kalium-, Natrium- und Triethanol-aminsalze.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen. Im Einzelnen ergeben sich die folgende Vorteile:
- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.
- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.
- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisations-vorgänge, Ausfällungen und Agglomeratbildung vermieden werden, da die Wechselwirkung unterbunden wird

Daher ist es bevorzugt, wenn ein oder mehrere der oben genannten UV-Filter in verkapselter Form vorliegen Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in genngem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Besonders bevorzugt einzusetzende Kapseln weisen Wände auf, die durch einen SolGel-Prozess, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Silicium-oxid-hydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäß einzusetzenden Zubereitungen vorzugsweise in solchen Mengen enthalten, die gewährleisten, dass die verkapselten UV-Filter in den oben angegebenen Mengen in der Zubereitung vorliegen

Besonders bevorzugte Wirkstoffe, insbesondere für hautpflegende Zubereitungen, sind beispielsweise auch sogenannte kompatible Solute Es handelt sich dabei um Substanzen, die an der Osmoregulation von Pflanzen oder Mikroorganismen beteiligt sind und aus diesen Organismen isoliert werden können Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin- Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden in den beschriebenen Selbstbräunungszubereitungen als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myo-inositol-phosphat (DIP), cyclisches 2,3-diphosphoglycerat (cDPG), 1,1- Diglycerin-Phosphat (DGP), ß-Mannosylglycerat (Firoin), ß- Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure) und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-ups, Pflegecremes und Sonnenschutzpräparaten eingesetzt werden.

Dabei wird vorzugsweise eine Pyrimidincarbonsäure gemäß der folgenden Formel eingesetzt, 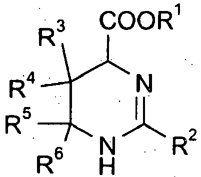 worin R¹ ein Rest H oder C1-8-Alkyl, R² ein Rest H oder C1-4-Alkyl und R³, R⁴, R⁵ sowie R⁶ jeweils unabhängig voneinander ein Rest aus der Gruppe H, OH, NH₂ und C1-4-Alkyl sind. Bevorzugt werden Pyrimidincarbonsäuren eingesetzt, bei denen R² eine Methyl- oder eine Ethylgruppe ist und R¹ bzw. R⁵ und R⁶ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäß einzusetzenden Zubereitungen derartige Pyrimidincarbonsäuren vorzugsweise in Mengen bis zu 15 Gew.-%.

Insbesondere bevorzugt ist es dabei, wenn die kompatiblen Solute ausgewählt sind aus Di-myo-inositol-phosphat (DIP), cyclisches 2,3-diphosphoglycerat (cDPG), 1,1- Diglycerin-Phosphat (DGP), ß-Mannosylglycerat (Firoin), ß-Mannosylglyceramid (Firoin-A) oder/und Di-mannosyl-di-inositolphosphat (DMIP), Ectoin, Hydroxyectoin oder Mischungen davon.

Unter den ebenfalls bevorzugt eingesetzten Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die zusätzlich eine Aryloxim, vorzugsweise 2-Hydroxy-5-methyllaurophenonoxim enthalten, zeigen antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew-% Aryloxim enthält.

Das mindestens eine Flavonoid nach Anspruch 1 kann auf übliche Weise, beispielsweise durch Mischen, in die Zubereitungen eingearbeitet werden.

Als Anwendungsform der einzusetzenden Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Bevorzugte Anwendungsformen sind auch Shampoos, Sonnenbäder und Duschbäder, die auch als sog. "Spray Tanning, Airbrush Tanning oder Sonnenduschen "aus kommerziellen Selbstbräunungs-Studios bekannt sind.

Bevorzugte Hilfsstoffe stammen aus der Gruppe der Konservierungsstoffe, Antioxidantien, Stabilisatoren, Lösungsvermittler, Vitamine, Färbemittel, Geruchs¬ verbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Iso-stearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche ÖJe wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsol, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen Es kann auch gegebenenfalls vorteilhaft sein, Wachse, beispielsweise Cetylpalmitat, als alleinige Lipidkomponente der Ölphase einzusetzen.

Die wässnge Phase der einzusetzenden Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z B Hyaluronsäure, Xanthangummi, Hydroxypropylmethyl-cellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet Bei alkoholischen Lösemitteln kann Wasser ein weiterer Bestandteil sein

Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

In einer bevorzugten Ausführungsform enthalten die einzusetzenden Zubereitungen hydrophile Tenside. Die hydrophilen Tenside werden bevorzugt gewählt aus der Gruppe der Alkylglucoside, der Acyllactylate, der Betaine sowie der Cocoamphoacetate

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren^{®} 1200 (Henkel KGaA), Oramix^{®} NS 10 (Seppic).

Die Zubereitung kann weiter Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Wachse, Lanolin, Treibmittel, Farbstoffe und/oder Pigmente, welche das Mittel selbst oder die Haut färben, und andere in der Kosmetik gewöhnlich verwendete Ingredienzien.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane , bevorzugt Alkane.

Die einzusetzenden Zubereitungen können dabei mit Hilfe von Techniken hergestellt werden, die dem Fachmann wohl bekannt sind.

Auch ohne weitere Ausführungen wird davon ausgegangen, dass ein Fachmann die obige Beschreibung in weitestem Umfang nutzen kann. Die bevorzugten Ausführungsformen sind deswegen lediglich als beschreibende, keinesfalls als in irgendeiner Weise limitierende Offenbarung aufzufassen. Die vollständige Offenbarung aller vor- und nachstehend aufgeführten Anmeldungen und Veröffentlichungen sind durch Bezugnahme in diese Anmeldung eingeführt.

Die im folgenden angeführten Beispiele für den erfindungsgemäßen Gegenstand dienen lediglich der Erläuterung und engen die vorliegende Erfindung keineswegs in irgendeiner Weise ein. Im übrigen ist die beschriebene Erfindung im gesamten beanspruchten Bereich ausführbar. Alle Verbindungen oder Komponenten, die in den Zubereitungen verwendet werden können, sind entweder bekannt und käuflich erhältlich oder können nach bekannten Methoden synthetisiert werden. Es werden die INCI-Namen der verwendeten Rohstoffe angegeben (die INCI-Namen werden definitionsgemäß in englischer Sprache angegeben).

### Beispiel 1 nicht erfindungsgemäß: Stabilisierung von Vanillin durch Troxerutin (Modell für eine wässrig-ethanolische Phase eines Emulsionssystems)

Durchführung: Es werden 10 mg Vanillin mit (Versuch A) bzw. ohne (Versuch B) 100 mg Troxerutin in Wasser/Ethanol (50/50) in 10 ml Messkolben gelöst und auf exakt 10 ml aufgefüllt. Anschließend wird in ein 20 ml Schraubglas umgefüllt (HPLC-Probe: 100 ml auf 1000 ml aufgefüllt) und für 96 Std. offen stark gerührt. Die Proben werden in die Messkolben zurück gespült, mit Ethanol nachgespült und dabei auf exakt 10 ml aufgefüllt. Die Auswertung per HPLC erfolgt nach den Peak-Ftächen (emeut 100 ml-Probe).

| **A (Troxerutin** + **Vanillin)** | | |
|---|---|---|
| **t[h]** | **HPLC-2** | |
| | Fläche | Vanillin* |
| 0 | 862860 | 100% |
| 96 | 840319 | 97% |

| **B (Vanillin)** | | |
|---|---|---|
| **t[h]** | **HPLC-2** | |
| | Fläche | Vanillin* |
| 0 | 897239 | 100% |
| 96 | 689849 | 77% |

Nach Versuchsdurchführung werden unstabilisiert 77 Gew.-% des ursprünglich eingesetzten Vanilin wiedergefunden, während in Gegenwart von Troxerutin, 97 Gew.-% wiedergefunden werden.

### Zubereitungen

Im folgenden werden beispielhaft Rezepturen für kosmetische Zubereitungen angegeben, die ein Flavonoid z.B. Rutin oder Troxerutin und eine Selbstbräunungssubstanz z.B. DHA enthalten. Im übrigen sind die INCI-Bezeichnungen der handelsüblichen Verbindungen angegeben. Die Herstellung der DHA-Duschlösungen erfolgt, indem man alle Rohstoffe einwiegt und homogen verrührt.

### Beispiel 2: DHA-Duschlösung

| **INCI** | **[Gew.-%]** |
|---|---|
| Aqua (Water) | 75.05 |
| Dihydroxyacetone | 8.00 |
| Rutin | 0.75 |
| Ectoin | 0.30 |
| Propylene glycol | 4.50 |
| Glycerin | 2.00 |
| Ethoxydiglycol | 5.00 |
| Dimethyl isosorbide | 2.00 |
| Polysorbate 80 | 0.50 |
| Propylene glycol, walnut extract | 1.50 |
| Caramel | 0.10 |
| Parfum | 0.30 |

### Beispiel 3: DHA-Duschlösung

Zusammensetzung analog zu Beispiel 2 , nur anstelle von Rutin wird 0.75% Troxerutin eingesetzt.

### Beispiel 4: Selbstbräunungscreme mit Flavonoid (O/W)

Die Herstellung der Selbstbräunungscreme -erfolgt indem man Phase A (bestehend aus Glycerylstearat, Stearylalkohol, Ceterarylalkohol, Cetearylethylhexanoat, Capryltriglycerid, Stearoxydimethicon, Dimethicon, Tocopherylacetat, Propylparaben) und Phase B (bestehend aus Propylenglykol, Methylparaben und Wasser) erhitzt. Phase B wird langsam in Phase A eingerührt und homogenisiert. Unter Rühren wird abgekühlt. Das Rutin vor dem DHA im Wasser lösen. Bei 40 °C wird Phase C (bestehend aus DHA, Rutin und Wasser) zugegeben.

| **INCI** | **[Gew.-%]** |
|---|---|
| Aqua (Water) | 69.00 |
| Dihydroxyacetone | 2.00 |
| Rutin | 1.00 |
| Methylparaben | 0.15 |
| Propylene glycol | 3.00 |
| Glyceryl stearate, Stearyl alcohol CETEH-20, STEARETH-25 | 8.00 |
| Cetearyl alcohol | 1.50 |
| Cetearyl ethylhexanoate | 6.50 |
| Caprylic/Capric triglycende | 6.50 |
| Stearoxy dimethicone | 1.20 |
| Dimethicone | 0.50 |
| Tocopheryl acetate | 0.50 |
| Propylparaben | 0.05 |
| Parfum | 0.10 |

### Beispiel 5: Selbstbräunungscreme mit Flavonoid (O/W)

Zusammensetzung analog zu Beispiel 4 , nur anstelle von Rutin wird 1 % Troxerutin eingesetzt.

### Beispiel 6: Selbstbräunungscreme mit Flavonoid (ONV)

| **INCI** | **[Gew.-%]** |
|---|---|
| Aqua (Water) | Ad 100 |
| Dihydroxyacetone | 0.75 |
| Troxerutin | 0.5 |
| Methyparaben | 0.15 |
| Propylene glycol | 3,00 |
| Glyceryl stearate, Stearyl alcohol CETEH-20, STEARETH-25 | 8.00 |
| Cetearyl alcohol | 1.50 |
| Cetearyl ethylhexanoate | 650 |
| Caprylic/Capric triglyceride | 6.50 |
| Stearoxy dimethicone | 1.20 |
| Dimethicone | 0.50 |
| Tocopheryl acetate | 0.50 |
| Propylparaben | 0.05 |
| Parfum | 0.10 |

### Vergleichsbeispiel 6V: Creme nach Beispiel 6, jedoch ohne Troxerutin

### Beispiel 7: Geruchsveränderung bei Lagerung

Die Cremes nach Beispiel 6 bzw. 6V werden 1 Jahr bei Raumtemperatur in geschlossenen Tiegeln gelagert. Nach dem Jahr werden beide Rezepturen zusätzlich frisch angesetzt. Die sensorische Prüfung zeigt, dass 6V sich im Geruch im Vergleich zu der frischen Zubereitung deutlich verändert hat, der Ursprungsgeruch ist weitgehend verloren, während der Geruch von Beispiel 6 sich in dem Jahr kaum verändert.

### Beispiel 8: Geruch auf der Haut

Die Cremes nach Beispiel 6 bzw. 6V werden frisch zubereitet auf die menschliche Haut aufgetragen und der Geruch direkt nach Auftragung sowie einige Stunden nach Auftragung sensorisch beurteilt.

Direkt nach Auftragung wird der Geruch der mit den Cremes behandelten Hautstellen bei beiden Cremes als angenehm empfunden. Einige Stunden später wird von den Testpersonen nur noch der mit Beispiel 6 behandelten Hautpartien als angenehm - leicht blumig bzw. vanilleartig - beschrieben, während der Geruch, der mit dem Vergleichsbeispiel 6V behandelten Hautpartien, von den Testpersonen als unangenehm bzw. als ähnlich dem Geruch von Liebstöckel beschrieben wird.

### Beispiel 9: DHA-Duschlotion

| **INCI** | **WY-02-01 %** | **WY-02-02 %** | **WY-02-03 %** | **WY-02-04 %** | **WY-02-05 %** | **WY-02-06 %** |
|---|---|---|---|---|---|---|
| Dihydroxyacetone | **8,00** | **10,00** | **8,00** | **10,00** | | |
| Dihydroxyacetone, Sodium Metabisulfite, Magnesium stearate | | | | | **8,00** | **10,00** |
| Sodium Metabisulfite | **0,80** | **1,00** | **0,80** | **1,00** | | |
| Troxerutin | **1,00** | **2,00** | **1,00** | **2,00** | **1,00** | **2,00** |
| Colorona® Bronze | | | **2,00** | **3,00** | | |
| Erythrulose | | | | | | |
| Ectoin | 0,50 | 0,50 | 0,50 | 0,50 | 0,50 | 0,50 |
| Propylene Glycol | 6,00 | 6,00 | 6,00 | 6,00 | 6,00 | 6,00 |
| Ethoxydiglycol | 5,00 | 5,00 | 5,00 | 5,00 | 5,00 | 5,00 |
| Glycenn | 4,00 | 4,00 | 4,00 | 4,00 | 4,00 | 4,00 |
| Dimethyl Isosorbide | 2,00 | 2,00 | 2,00 | 2,00 | 2,00 | 2,00 |
| Polysorbate 80 | 1,60 | 1,60 | 1,60 | 1,60 | 1,60 | 1,60 |
| Aloe Barbadensis | 0,30 | 0,30 | 0,30 | 0,30 | 0,30 | 0,30 |
| Colorants | q.s | q.s. | q s | q s | q s. | q.s. |
| Preservative | q.s. | q.s | . q.s. | q.s. | q.s. | q.s. |
| Aqua (Water) | 70,80 | 67,60 | 68,80 | 64,60 | 71,60 | 68,60 |
| **ad** | **100,00** | **100,00** | **100,00** | **100,00** | **100,00** | **100,00** |

| **INCI** | **WY-02-07 %** | **WY-02-08 %** | **WY-02-09 %** | **WY-02-10 %** | **WY-02-11 %** | **WY-02-12 %** |
|---|---|---|---|---|---|---|
| Dihydroxyacetone | | | **8,00** | **10,00** | **10,00** | |
| Dihydroxyacetone, Sodium Metabisulfite, Magnesium stearate | **8,00** | **10,00** | | | | **8,00** |
| Sodium Metabisulfite | | | **0,80** | **1,00** | **1,00** | |
| Troxerutin | **1,00** | **2,00** | **1,00** | **2,00** | **2,00** | **1,00** |
| Colorona® Bronze | **2,00** | **3,00** | | | **3,00** | |
| Erythrulose | | | 3,00 | 3,50 | 3,50 | 3,00 |
| Ectoin | 0,50 | 0,50 | 0,50 | 0,50 | 0,50 | 0,50 |
| Propylene Glycol | 6,00 | 6,00 | 6,00 | 6,00 | 6,00 | 6,00 |
| Ethoxydiglycol | 5,00 | 5,00 | 5,00 | 5,00 | 5,00 | 5,00 |
| Glycenn | 4,00 | 4,00 | 4,00 | 4,00 | 4,00 | 4,00 |
| Dimethyl Isosorbide | 2,00 | 2,00 | 2,00 | 2,00 | 2,00 | 2,00 |
| Polysorbate 80 | 1,60 | 1,60 | 1,60 | 1,60 | 1,60 | 1,60 |
| Aloe Barbadensis | 0,30 | 0,30 | 0,30 | 0,30 | 0,30 | 0,30 |
| Colorants | q.s. | q.s | q.s. | q.s | q.s. | q.s. |
| Preservative | q.s. | q.s. | q.s. | q.s. | q.s. | q.s. |
| Aqua (Water) | 69,60 | 65,60 | 67,80 | 64,10 | 61,10 | 68,60 |
| **Komplett** | **100,00** | **100,00** | **100,00** | **100,00** | **100,00** | **100,00** |

| **INCI** | **WY-02-13 %** | **WY-02-14 %** | **WY-02-15 %** | **WY-02-16 %** | **WY-02-17 %** | **WY-02-18 %** |
|---|---|---|---|---|---|---|
| Dihydroxyacetone | | | **8,00** | **10,00** | **10,00** | |
| Dihydroxyacetone, Sodium Metabisulfite, Magnesium stearate | **10,00** | **10,00** | | | | **8,00** |
| Sodium Metabisulfite | | | | **1,00** | **1,00** | |
| Troxerutin | **2,00** | **2,00** | **1,00** | | | |
| Rutinsulfat | | | | **2,00** | **2,00** | **1,00** |
| Colorona® Bronze | | 3,00 | | | **3,00** | |
| Erythrulose | 3,50 | 3,50 | 3,00 | 3,50 | 3,50 | 3,00 |
| Ectoin | 0,50 | 0,50 | 0,50 | 0,50 | 0,50 | 0,50 |
| Propylene Glycol | 6,00 | 6,00 | 6,00 | 6,00 | 6,00 | 6,00 |
| Ethoxydiglycol | 5,00 | 5,00 | 5,00 | 5,00 | 5,00 | 5,00 |
| Glycenn | 4,00 | 4,00 | 4,00 | 4,00 | 4,00 | 4,00 |
| Dimethyl Isosorbide | 2,00 | 2,00 | 2,80 | 2,00 | 2,00 | 2,00 |
| Polysorbate 80 | 1,60 | 1,60 | 1,60 | 1,60 | 1,60 | 1,60 |
| Aloe Barbadensis | 0,30 | 0,30 | 0,30 | 0,30 | 0,30 | 0,30 |
| Colorants | q.s. | q.s. | q.s. | q.s. | q.s. | q.s. |
| Preservative | q.s. | q.s | . q.s. | q.s. | q.s. | q.s. |
| Aqua (Water) | 65,10 | 62,10 | 66,80 | 64,10 | 61,10 | 68,60 |
| **Komplett** | **100,00** | **100,00** | **100,00** | **100,00** | **100,00** | **100,00** |

Prozeß: Alle Rohstoffe werden zur Homogenität gerührt.

| **INCI** | **WY-02-28 %** | **WY-02-29 %** | **WY-02-30 %** | **WY-02-31 %** |
|---|---|---|---|---|
| Dihydroxyacetone | **8,00** | **10,00** | **8,00** | **10,00** |
| Troxerutin | **2,00** | **3,00** | **2,00** | **3,00** |
| Colorona® Bronze | **3,00** | **5,00** | **3,00** | **5,00** |
| Erythrulose | | | **3,00** | **5,00** |
| Ectoin | 0,50 | 0,50 | 0,50 | 0,50 |
| Propylene Glycol | 6,00 | 6,00 | 6,00 | 6,00 |
| Ethoxydiglycol | 5,00 | 5,00 | 5,00 | 5,00 |
| Glycerin | 4,00 | 4,00 | 4,00 | 4,00 |
| Dimethyl Isosorbide | 2,00 | 2,00 | 2,00 | 2,00 |
| Polysorbate 80 | 1,60 | 1,60 | 1,60 | 1,60 |
| Aloe Barbadensis | 0,30 | 0,30 | 0,30 | 0,30 |
| Colorants | q.s. | q.s. | q.s. | q.s. |
| Preservative | q.s. | q.s. | q.s. | q.s. |
| Aqua (Water) | 67,60 | 62,60 | 64,60 | 57,60 |
| **ad** | **100,00** | **100,00** | **100,00** | **100,00** |

Prozeß Alle Rohstoffe werden zur Homogenität gerührt

### Beispiel 10: DHA-Duschlotion

| **INCI** | **WY-01-01 %** | **WY-01-02 %** | **WY-01-03 %** | **WY-01-04 %** | **WY-01-05 %** | **WY-01-06 %** |
|---|---|---|---|---|---|---|
| Dihydroxyacetone | **8,00** | **10,00** | **8,00** | **10,00** | | |
| Dihydroxyacetone, Sodium Metabisulfite, Magnesium stearate | | | | | **8,00** | **10,00** |
| Sodium Metabisulfite | **0,80** | **1,00** | **0,80** | **1,00** | | |
| Troxerutin | **1,00** | **2,00** | **1,00** | **2,00** | **1,00** | **2,00** |
| Colorona® Bronze | | | **2,00** | **3,00** | | |
| Erythrulose | | | | | | |
| Ectoin | 0,50 | 0,50 | 0,50 | 0,50 | 0,50 | 0,50 |
| Propylene Glycol | 4,50 | 4,50 | 4,50 | 4,50 | 4,50 | 4,50 |
| Glycerin | 4,00 | 4,00 | 4,00 | 4,00 | 4,00 | 4,00 |
| Ethoxydiglycol | 3,00 | 3,00 | 3,00 | 3,00 | 3,00 | 3,00 |
| Dimethyl Isosorbide | 2,00 | 2,00 | 2,00 | 2,00 | 2,00 | 2,00 |
| Polysorbate 80 | 0,50 | 0,50 | 0,50 | 0,50 | 0,50 | 0,50 |
| Walnut Extract, Propylene Glycol | | 1,50 | 1,50 | 1,50 | 1,50 | 1,50 |
| Caramel | 0,10 | 0,10 | 0,10 | 0,10 | 0,10 | 0,10 |
| Parfum | 0,30 | 0,30 | 0,30 | 0,30 | 0,30 | 0,30 |
| Phenoxyethanol, Butyl- araben, Ethylparaben, propylparaben, Methyl-araben | 0,80 | 0,80 | 0,80 | 0,80 | 0,80 | 0,80 |
| Aqua (Water) | 73,00 | 69,80 | 71,00 | 66,80 | 73,80 | 68,60 |
| **Komplett** | **100,00** | **100,00** | **100,00** | **100,00** | **100,00** | **100,00** |

Prozeß: Alle Rohstoffe werden zur Homogenität gerührt.

| **INCI** | **WY-01-07 %** | **WY-01-08 %** | **WY-01-09 %** | **WY-01-10 %** | **WY-01-11 %** | **WY-01-12 %** |
|---|---|---|---|---|---|---|
| Dihydroxyacetone | | | **8,00** | **10,00** | **10,00** | |
| Dihydroxyacetone, Sodium Metabisulfite, Magnesium stearate | | | | | | **8,00** |
| Sodium Metabisulfite | | | **0,80** | **1,00** | **1,00** | |
| Troxerutin | **1,00** | **2,00** | **1,00** | **2,00** | **2,00** | **1,00** |
| Colorona® Bronze | **2,00** | **3,00** | | | **3,00** | |
| Erythrulose | | | **3,00** | **3,50** | **3,50** | **3,00** |
| Ectoin | 0,50 | 0,50 | 0,50 | 0,50 | 0,50 | 0,50 |
| Propylene Glycol | 4,50 | 4,50 | 4,50 | 4,50 | 4,50 | 4,50 |
| Glycerin | 4,00 | 4,00 | 4,00 | 4,00 | 4,00 | 4,00 |
| Ethoxydiglycol | 3,00 | 3,00 | 3,00 | 3,00 | 3,00 | 3,00 |
| Dimethyl Isosorbide | 2,00 | 2,00 | 2,00 | 2,00 | 2,00 | 2,00 |
| Polysorbate 80 | 0,50 | 0,50 | 0,50 | 0,50 | 0,50 | 0,50 |
| Walnut Extract, Propylene Glycol | 1,50 | 1,50 | 1,50 | 1,50 | 1,50 | 1,50 |
| Caramel | 0,10 | 0,10 | 0,10 | 0,10 | 0,10 | 0,10 |
| Parfum | 0,30 | 0,30 | 0,30 | 0,30 | 0,30 | 0,30 |
| Phenoxyethanol, Butyl- araben, Ethylparaben, propylparaben, Methyl-araben | 0,80 | 0,80 | 0,80 | 0,80 | 0,80 | 0,80 |
| Aqua (Water) | 71,80 | 67,80 | 70,00 | 66,30 | 63,30 | 70,80 |
| **Komplett** | **100,00** | **100,00** | **100,00** | **100,00** | **100,00** | **100,00** |

Prozeß: Alle Rohstoffe werden zur Homogenität gerührt.

| **INCI** | **WY-01-13 %** | **WY-01-14 %** | **WY-01-15 %** | **WY-01-16 %** |
|---|---|---|---|---|
| Dihydroxyacetone | **8,00** | **10,00** | **8,00** | **10,00** |
| Troxerutin | **2,00** | **3,00** | **2,00** | **3,00** |
| Colorona® Bronze | **3,00** | **5,00** | **3,00** | **5,00** |
| Erythrulose | | | **3,00** | **5,00** |
| Ectoin | 0,50 | 0,50 | 0,50 | 0,50 |
| Propylene Glycol | 4,50 | 4,50 | 4,50 | 4,50 |
| Glycerin | 4,00 | 4,00 | 4,00 | 4,00 |
| Ethoxydiglycol | 3,00 | 3,00 | 3,00 | 3,00 |
| Dimethyl Isosorbide | 2,00 | 2,00 | 2,00 | 2,00 |
| Polysorbate 80 | 0,50 | 0,50 | 0,50 | 0,50 |
| Walnut Extract, Propylene Glycol | 1,50 | 1,50 | 1,50 | 1,50 |
| Caramel | 0,10 | 0,10 | 0,10 | 0,10 |
| Parfum | 0,30 | 0,30 | 0,30 | 0,30 |
| Phenoxyethanol, Butyl- araben, Ethylparaben, Propylparaben, Methyl-araben | 0,80 | 0,80 | 0,80 | 0,80 |
| Aqua (Water) | 71,00 | 66,80 | 73,80 | 70,80 |
| **Komplett** | **101,20** | **102,00** | **107,00** | **111,00** |

Prozeß: Alle Rohstoffe werden zur Homogenität gerührt.

### Beispiel 11: Hydrogel enthaltend DHA

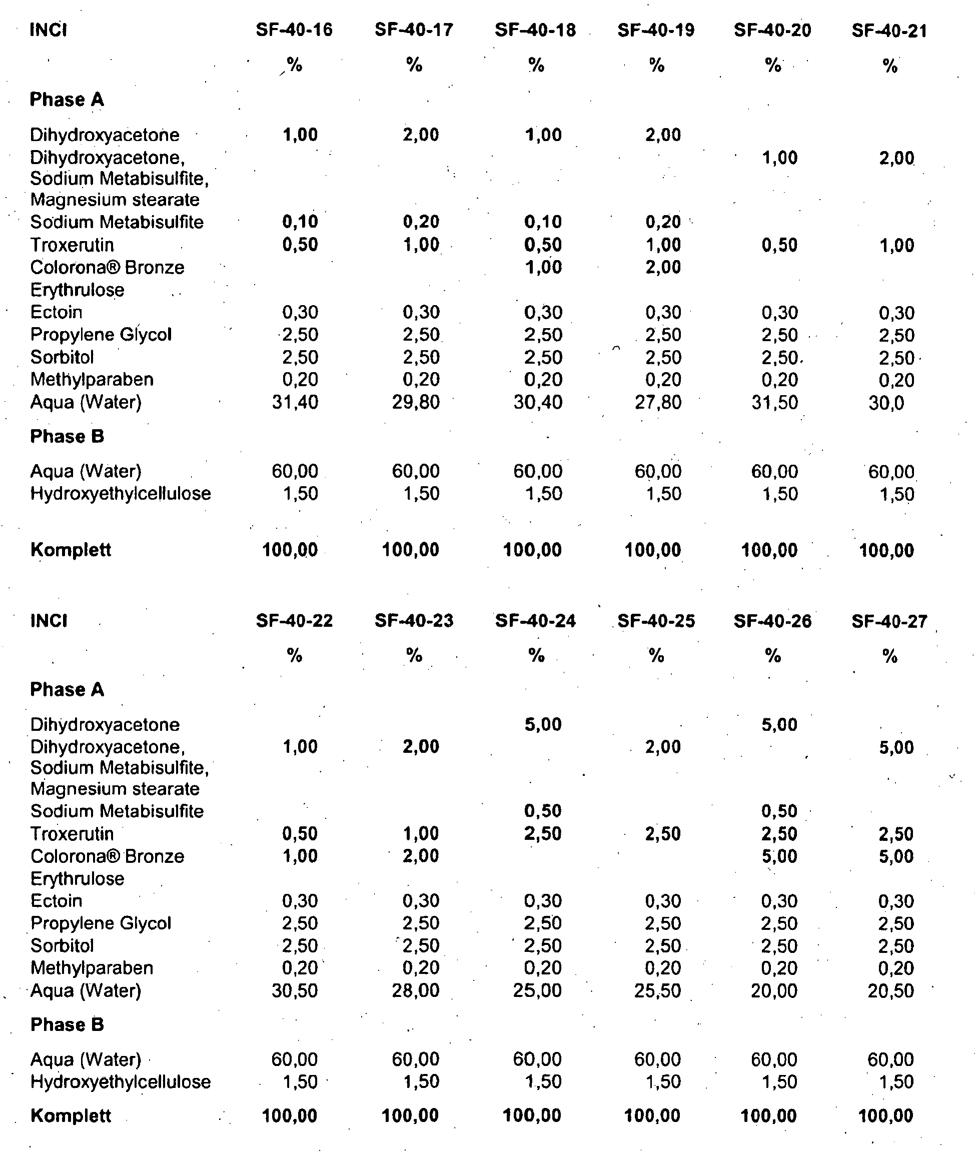

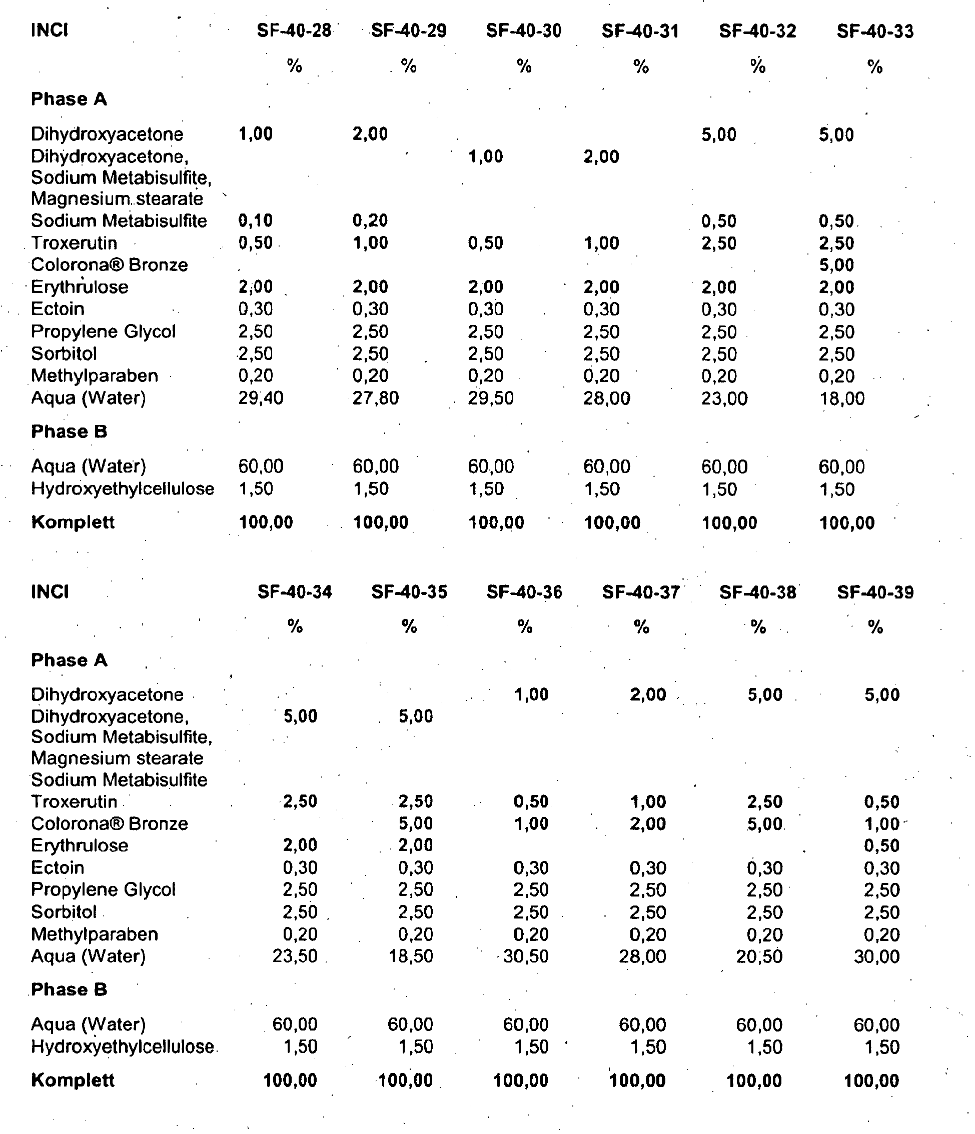

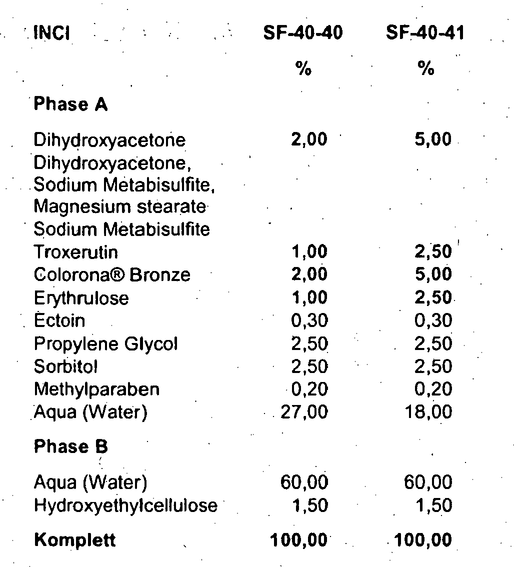

### Prozeß:

Hydroxyethylcellulose wird unter starkem Rühren zum Wasser der Phase B gegeben. Die Zugabe muss in einer Geschwindigkeit erfolgen, die es den Partikeln erlaubt, zu separieren und deren Oberfläche zu benetzen, jedoch muß darauf geachtet werden, dass die Viskosität minimiert wird.

Eine Aufbewahrung bei Raumtemperatur wird empfohlen.

### Beispiel 12: W/O Lotion mit Troxerutin

| **INCI** | **ST-16-42 %** | **ST-16-43 %** | **ST-16-44 %** | **ST-16-45 %** | **ST-16-46 %** | **ST-16-47 %** | **ST-16-48 %** |
|---|---|---|---|---|---|---|---|
| **Phase A** | | | | | | | |
| Bis-PEG/PPG-14/14 Dimethicone, Cyclopentasiloxane | 1,50 | 1,50 | 1,50 | 1,50 | 1,50 | 1,50 | 1,50 |
| Cetyl PEG/PPG-10/1 Dimethicone | 1,30 | 1,30 | 1,30 | 1,30 | 1,30 | 1,30 | 1,30 |
| Cyclomethicone | 13,00 | 13,00 | 13,00 | 13,00 | 13,00 | 13,00 | 13,00 |
| Ethylhexyl Palmitate | 2,00 | 2,00 | 2,00 | 2,00 | 2,00 | 2,00 | 2,00 |
| Cyclopentasiloxane, Dimethicone/Vinyl-dimethicone, Crosspolymer | 5,00 | 5,00 | 5,00 | 5,00 | 5,00 | 5,00 | 5,00 |
| Diethylhexyl Carbonate | 5,00 | 5,00 | 5,00 | 5,00 | 5,00 | 5,00 | 5,00 |
| Parfum | 0,30 | 0,30 | 0,30 | 0,30 | 0,30 | 0,30 | 0,30 |

| **Phase B** | | | | | | | |
|---|---|---|---|---|---|---|---|
| Dihydroxyacetone | **1,00** | **2,00** | **1,00** | **2,00** | | | |
| Dihydroxyacetone, Sodium Metabisulfite, Magnesium stearate | | | | | **1,00** | **2,00** | **1,00** |
| Sodium Metabisulfite | **0,10** | **0,20** | **0,10** | **0,20** | | | |
| Troxerutin | **0,50** | **1,00** | **0,50** | **1,00** | **0,50** | **1,00** | **0,50** |
| Colorona® Bronze | | | **1,00** | **2,00** | | | |
| Erythrulose Ectoin | 0,30 | 0,30 | 0,30 | 0,30 | 0,30 | 0,30 | 0,30 |
| Propylene Glycol | 18,00 | 18,00 | 18,00 | 18,00 | 18,00 | 18,00 | 18,00 |
| Glycenn | 3,00 | 3,00 | 3,00 | 3,00 | 3,00 | 3,00 | 3,00 |
| Magnesium Sulfate | 2,00 | 2,00 | 2,00 | 2,00 | 2,00 | 2,00 | 2,00 |
| Alcohol | 8,00 | 8,00 | 8,00 | 8,00 | 8,00 | 8,00 | 8,00 |
| Phenoxyethanol, Butylparaben, Ethylparaben, Propylparaben, Methylparaben | 1,00 | 1,00 | 1,00 | 1,00 | 1,00 | 1,00 | 1,00 |
| Aqua (Water) | 38,00 | 36,40 | 37,00 | 34,40 | 38,10 | 36,60 | 37,10 |
| **Complete** | **100,00** | **100,00** | **100,00** | **100,00** | **100,00** | **100,00** | **100,00** |

| **INCI** | **ST-16-49 %** | **ST-16-50 %** | **ST-16-51 %** | **ST-16-52 %** | **ST-16-53 %** | **ST-16-54 %** | **ST-16-55 %** |
|---|---|---|---|---|---|---|---|
| **Phase A** | | | | | | | |
| Bis-PEG/PPG-14/14 Dimethicone, Cyclopentasiloxane | 1,50 | 1,50 | 1,50 | 1,50 | 1,50 | 1,50 | 1,50 |
| Cetyl PEG/PPG-10/1 Dimethicone | 1,30 | 1,30 | 1,30 | 1,30 | 1,30 | 1,30 | 1,30 |
| Cyclomethicone | 13,00 | 13,00 | 13,00 | 13,00 | 13,00 | 13,00 | 13,00 |
| Ethylhexyl Palmitate | 2,00 | 2,00 | 2,00 | 2,00 | 2,00 | 2,00 | 2,00 |
| Cyclopentasiloxane, Dimethicone/Vinyl-dimethicone, Crosspolymer | 5,00 | 5,00 | 5,00 | 5,00 | 5,00 | 5,00 | 5,00 |
| Diethylhexyl Carbonate | 5,00 | 5,00 | 5,00 | 5,00 | 5,00 | 5,00 | 5,00 |
| Parfum | 0,30 | 0,30 | 0,30 | 0,30 | 0,30 | 0,30 | 0,30 |

| **Phase B** | | | | | | | |
|---|---|---|---|---|---|---|---|
| Dihydroxyacetone | | **5,00** | | **5,00** | | **1,00** | **2,00** |
| Dihydroxyacetone, Sodium Metabisulfite, Magnesium stearate | **2,00** | | **5,00** | | **5,00** | | |
| Sodium Metabisulfite | | **0,50** | | **0,50** | | **0,10** | **0,20** |
| Troxerutin | **1,00** | **2,50** | **2,50** | **2,50** | **2,50** | **0,50** | **1,00** |
| Colorona® Bronze | **2,00** | | | **5,00** | **5,00** | | |
| Erythrulose | | | | | | 2,00 | 2,00 |
| Ectoin | 0,30 | 0,30 | 0,30 | 0,30 | 0,30 | 0,30 | 0,30 |
| Propylene Glycol | 18,00 | 18,00 | 18,00 | 18,00 | 18,00 | 18,00 | 18,00 |
| Glycerin | 3,00 | ' 3,00 | 3,00 | 3,00 | 3,00 | 3,00 | 3,00 |
| Magnesium Sulfate | 2,00 | 2,00 | 2,00 | 2,00 | 2,00 | 2,00 | 2,00 |
| Alcohol | 8,00 | 8,00 | 8,00 | 8,00 | 8,00 | 8,00 | 8,00 |
| Phenoxyethanol, Butylparaben, Ethylparaben, Propylparaben, Methylparaben | 1,00 | 1,00 | 1,00 | 1,00 | 1,00 | 1,00 | 1,00 |
| Aqua (Water) | 34,60 | 31,60 | 32,10 | 26,60 | 27,10 | 36,00 | 34,40 |
| **Complete** | **100,00** | **100,00** | **100,00** | **100,00** | **100,00** | **100,00** | **100,00** |

| **INCI** | **ST-16-56 %** | **ST-16-57 %** | **ST-16-58 %** | **ST-16-59 %** | **ST-16-60 %** | **ST-16-61 %** | **ST-16-62 %** |
|---|---|---|---|---|---|---|---|
| **Phase A** | | | | | | | |
| Bis-PEG/PPG-14/14 Dimethicone, Cyclopentasiloxane | 1,50 | 1,50 | 1,50 | 1,50 | 1,50 | 1,50 | 1,50 |
| Cetyl PEG/PPG-10/1 Dimethicone | 1,30 | 1,30 | 1,30 | 1,30 | 1,30 | 1,30 | 1,30 |
| Cyclomethicone | 13,00 | 13,00 | 13,00 | 13,00 | 13,00 | 13,00 | 13,00 |
| Ethylhexyl Palmitate | 2,00 | 2,00 | 2,00 | 2,00 | 2,00 | 2,00 | 2,00 |
| Cyclopentasiloxane, Dimethicone/Vinyldimethicone, Crosspolymer | 5,00 | 5,00 | 5,00 | 5,00 | 5,00 | 5,00 | 5,00 |
| Diethylhexyl Carbonate | 5,00 | 5,00 | 5,00 | 5,00 | 5,00 | 5,00 | 5,00 |
| Parfum | 0,30 | 0,30 | 0,30 | 0,30 | 0,30 | 0,30 | 0,30 |

| **Phase B** | | | | | | | |
|---|---|---|---|---|---|---|---|
| Dihydroxyacetone | | | **5,00** | **5,00** | | | **1,00** |
| Dihydroxyacetone, Sodium Metabisulfite, Magnesium stearate | **1,00** | **2,00** | | | **5,00** | **5,00** | |
| Sodium Metabisulfite | | | **0,50** | **0,50** | | | |
| Troxerutin | **0,50** | **1,00** | **2,50** | **2,50** | **2,50** | **2,50** | **0,50** |
| Colorona® Bronze | | | | **5,00** | | **5,00** | **1,00** |
| Erythrulose | **2,00** | **2,00** | **2,00** | **2,00** | **2,00** | **2,00** | |
| Ectoin | 0,30 | 0,30 | 0,30 | 0,30 | 0,30 | 0,30 | 0,30 |
| Propylene Glycol | 18,00 | 18,00 | 18,00 | 18,00 | 18,00 | 18,00 | 18,00 |
| Glycerin | 3,00 | 3,00 | 3,00 | 3,00 | 3,00 | 3,00 | 3,00 |
| Magnesium Sulfate | 2,00 | 2,00 | 2,00 | 2,00 | 2,00 | 2,00 | 2,00 |
| Alcohol | 8,00 | 8,00 | 8,00 | 8,00 | 8,00 | 8,00 | 8,00 |
| Phenoxyethanol, Butylparaben, Ethylparaben, Propylparaben, Methylparaben | 1,00 | 1,00 | 1,00 | 1,00 | 1,00 | 1,00 | 1,00 |
| Aqua (Water) | 36,10 | 34,60 | 29,60 | 24,60 | 30,10 | 25,10 | 37,10 |
| | | | | | | | |
| **Complete** | **100,00** | **100,00** | **100,00** | **100,00** | **100,00** | **100,00** | **100,00** |

| **INCI** | **ST-16-63 %** | **ST-16-64 %** | **ST-16-65 %** | **ST-16-66 %** | **ST-16-67 %** |
|---|---|---|---|---|---|
| **Phase A** | | | | | |
| Bis-PEG/PPG-14/14 Dimethicone, Cyclopentasiloxane | 1,50 | 1,50 | 1,50 | 1,50 | 1,50 |
| Cetyl PEG/PPG-10/1 Dimethicone | 1,30 | 1,30 | 1,30 | 1,30 | 1,30 |
| Cyclomethicone | 13,00 | 13,00 | 13,00 | 13,00 | 13,00 |
| Ethylhexyl Palmitate | 2,00 | 2,00 | 2,00 | 2,00 | 2,00 |
| Cyclopentasiloxane, Dimethicone/Vinyl-dimethicone, Crosspolymer | 5,00 | 5,00 | 5,00 | 5,00 | 5,00 |
| Diethylhexyl Carbonate | 5,00 | 5,00 | 5,00 | 5,00 | 5,00 |
| Parfum | 0,30 | 0,30 | 0,30 | 0,30 | 0,30 |

| **Phase B** | | | | | |
|---|---|---|---|---|---|
| Dihydroxyacetone | **2,00** | **5,00** | **1,00** | **2,00** | **5,00** |
| Dihydroxyacetone, Sodium Metabisulfite, | | | | | |
| Magnesium stearate Sodium Metabisulfite | | | | | **5,00** |
| Troxerutin | **1,00** | **2,50** | **0,50** | **1,00** | **2,50** |
| Colorona® Bronze | **2,00** | **5,00** | **1,00** | **2,00** | **5,00** |
| Erythrulose | | | **0,50** | **1,00** | **2,50** |
| Ectoin | 0,30 | 0,30 | 0,30 | 0,30 | 0,30 |
| Propylene Glycol | 18,00 | 18,00 | 17,00 | 17,00 | 17,00 |
| Glycerin | 3,00 | 3,00 | 3,00 | 3,00 | 3,00 |
| Magnesium Sulfate | 2,00 | 2,00 | 2,00 | 2,00 | 2,00 |
| Alcohol | 8,00 | 8,00 | 8,00 | 8,00 | 8,00 |
| Phenoxyethanol, Butylparaben, Ethylparaben, Propylparaben, Methylparaben | 1,00 | 1,00 | 1,00 | 1,00 | 1.00 |
| Aqua (Water) | 34,60 | 27,10 | 37,60 | 34,60 | 25,60 |
| | | | | | |
| **Complete** | **100,00** | **100,00** | **100,00** | **100,00** | **100,00** |

### Prozeß:

Zunächst wird Magnesium Sulfat im Wasser der Phase B gerührt und anschließend die weiteren Inhaltsstoffe der Phase B zugegeben. Phase B wird anschließend langsam unter Rühren zu Phase A zugegeben und homogenisiert.

### Beispiel 13: O/W Creme mit Troxerutin

| **INCI** | **ST-08-29 %** | **ST-08-30 %** | **ST-08-31 %** | **ST-08-32%** | **ST-08-33 %** | **ST-08-34 %** | **ST-08-35 %** |
|---|---|---|---|---|---|---|---|
| **Phase A** | | | | | | | |
| Glyeryl Stearate, Steareth-26, Ceteth-20, Stearyl Alcohol | 8,00 | 8,00 | 8,00 | 8,00 | 8,00 | 8,00 | 8,00 |
| Cetearyl Alcohol | 1,50 | 1,50 | 1,50 | 1,50 | 1,50 | 1,50 | 1,50 |
| Cetearyl Ethylhexanoate | 5,50 | 5,50 | 5,50 | 5,50 | 5,50 | 5,50 | 5,50 |
| Caprylic/Capric Triglyceride | 5,50 | 5,50 | 5,50 | 5,50 | 5,50 | 5,50 | 5,50 |
| Stearoxy Dimethicone | 1,00 | 1,00 | 1,00 | 1,00 | 1,00 | 1,00 | 1,00 |
| Dimethicone | 0,50 | 0,50 | 0,50 | 0,50 | 0,50 | 0,50 | 0,50 |
| Tocopheryl Acetate | 0,50 | 0,50 | .0,50 | 0,50 | 0,50 | 0,50 | 0,50 |
| Propylparaben | 0,05 | 0,05 | 0,05 | 0,05 | 0,05 | 0,05 | 0,05 |

| **Phase B** | | | | | | | |
|---|---|---|---|---|---|---|---|
| Ectoin | 0,30 | 0,30 | 0,30 | 0,30 | 0,30 | 0,30 | 0,30 |
| Propylene Glycol | 3,00 | 3,00 | 3,00 | 3,00 | 3,00 | 3,00 | 3,00 |
| Methylparaben | 0,15 | 0,15 | 0,15 | 0,15 | 0,15 | 0,15 | 0,15 |
| Aqua (Water) | 62,30 | 60,70 | 61,30 | 58,70 | 62,40 | 60,90 | 61,40 |

| **Phase C** | | | | | | | |
|---|---|---|---|---|---|---|---|
| Dihydroxyacetone | **1,00** | **2,00** | **1,00** | **2,00** | | | |
| Dihydroxyacetone, Sodium, Metabisulfite, Magnesium stearate | | | | | **1,00** | **2,00** | **1,00** |
| Sodium Metabisulfite | **0,10** | **0,20** | **0,10** | **0,20** | | | |
| Troxerutin | **0,50** | **1,00** | **0,50** | **1,00** | **0,50** | **1,00** | **0,50** |
| Colorona® Bronze | | | **1,00** | **2,00** | | | **1,00** |
| Erythrulose | | | | | | | |
| Aqua (Water) | 10,00 | 10,00 | 10,00 | 10,00 | 10,00 | 10,00 | 10,00 |

| **Phase D** | | | | | | | |
|---|---|---|---|---|---|---|---|
| Parfum | 0,10 | 0,10 | 0,10 | 0,10 | 0,10 | 0,10 | 0,10 |
| | | | | | | | |
| **Complete** | **100,00** | **100,00** | **100,00** | **100,00** | **100,00** | **100,00** | **100,00** |

| **INCI** | **ST-08·36 %** | **ST-08-37 %** | **ST-08-38 %** | **ST-08-39 %** | **ST-08-40 %** | **ST-08-41 %** | **ST-08-42 %** |
|---|---|---|---|---|---|---|---|
| **Phase A** | | | | | | | |
| Glyeryl Stearate, Steareth-26, Ceteth-20, Stearyl Alcohol | 8,00 | 8,00 | 8,00 | 8,00 | 8,00 | 8,00 | 8,00 |
| Cetearyl Alcohol | 1,50 | 1,50 | 1,50 | 1,50 | 1,50 | 1,50 | 1,50 |
| Cetearyl Ethylhexanoate | 5,50 | 5,50 | 5,50 | 5,50 | 5,50 | 5,50 | 5,50 |
| Caprylic/Capric Triglyceride | 5,50 | 5,50 | 5,50 | 5,50 | 5,50 | 5,50 | 5,50 |
| Stearoxy Dimethicone | 1,00 | 1,00 | 1,00 | 1,00 | 1,00 | 1,00 | 1,00 |
| Dimethicone | 0,50 | 0,50 | 0,50 | 0,50 | 0,50 | 0,50 | 0,50 |
| Tocopheryl Acetate | 0,50 | 0,50 | 0,50 | 0.50 | 0,50 | 0,50 | 0,50 |
| Propylparaben | 0,05 | 0,05 | 0,05 | 0,05 | 0,05 | 0,05 | 0,05 |

| **Phase B** | | | | | | | |
|---|---|---|---|---|---|---|---|
| Ectoin | 0,30 | 0,30 | 0,30 | 0,30 | 0,30 | 0,30 | 0,30 |
| Propylene Glycol | 3,00 | 3,00 | 3,00 | 3,00 | 3,00 | 3,00 | 3,00 |
| Methylparaben | 0,15 | 0,15 | 0,15 | 0,15 | 0,15 | 0,15 | 0,15 |
| Aqua (Water) | 58,90 | 55,90 | 56,40 | 50,90 | 51,40 | 60,30 | 58,70 |

| **Phase C** | | | | | | | |
|---|---|---|---|---|---|---|---|
| Dihydroxyacetone | | **5,00** | | **5,00** | | **1,00** | **2,00** |
| Dihydroxyacetone, Sodium, Metabisulfite Magnesium stearate | **2,00** | | **5,00** | | **5,00** | | |
| Sodium Metabisulfite | | **0,50** | | **0,50** | | **0,10** | **0,20** |
| Troxerutin | **1,00** | **2,50** | **2,50** | **2,50** | **2,50** | **0,50** | **1,00** |
| Colorona® Bronze | **2,00** | | | **5,00** | **5,00** | | |
| Erythrulose | | | | | | **2,00** | **2,00** |
| Aqua (Water) | 10,00 | 10,00 | 10,00 | 10,00 | 10,00 | 10,00 | 10.00 |

| **Phase D** | | | | | | | |
|---|---|---|---|---|---|---|---|
| Parfum | 0,10 | 0,10 | 0,10 | 0,10 | 0,10 | 0,10 | 0,10 |
| | | | | | | | |
| **Complete** | **100,00** | **100,00** | **100,00** | **100,00** | **100,00** | **100,00** | **100,00** |

| **INCI** | **ST-08-43 %** | **ST-08-44 %** | **ST-08-45 %** | **ST-08-46 %** | **ST-08-47 %** | **ST-08-48 %** | **ST-08-49 %** |
|---|---|---|---|---|---|---|---|
| **Phase A** | | | | | | | |
| Glyeryl Stearate, Steareth-26, Ceteth-20, Stearyl Alcohol | 8,00 | 8,00 | 8,00 | 8,00 | 8,00 | 8,00 | 8,00 |
| Cetearyl Alcohol | 1,50 | 1,50 | 1,50 | 1,50 | 1,50 | 1,50 | 1,50 |
| Cetearyl Ethylhexanoate | 5,50 | 5,50 | 5,50 | 5,50 | 5,50 | 5,50 | 5,50 |
| Caprylic/Capric Triglyceride | 5,50 | 5,50 | 5,50 | 5,50 | 5,50 | 5,50 | 5,50 |
| Stearoxy Dimethicone | 1,00 | 1,00 | 1.00 | 1,00 | 1,00 | 1,00 | 1,00 |
| Dimethicone | 0,50 | 0,50 | 0,50 | 0,50 | 0,50 | 0,50 | 0,50 |
| Tocopheryl Acetate | 0,50 | 0,50 | 0,50 | 0,50 | 0,50 | 0,50 | 0,50 |
| Propylparaben | 0,05 | 0,05 | 0,05 | 0,05 | 0,05 | 0,05 | 0,05 |

| **Phase B** | | | | | | | |
|---|---|---|---|---|---|---|---|
| Ectoin | 0,30 | 0,30 | 0,30 | 0,30 | 0,30 | 0,30 | 0,30 |
| Propylene Glycol | 3,00 | 3,00 | 3,00 | 3,00 | 3,00 | 3,00 | 3,00 |
| Methylparaben | 0,15 | 0,15 | 0,15 | 0,15 | 0,15 | 0,15 | 0,15 |
| Aqua (Water) | 60,40 | 58,90 | 53,90 | 48,990 | 54,40 | 49,40 | 61,40 |

| **Phase C** | | | | | | | |
|---|---|---|---|---|---|---|---|
| Dihydroxyacetone | | | **5,00** | **5,00** | | | **1,00** |
| Dihydroxyacetone, Sodium, Metabisulfite, Magnesium stearate | **1,00** | **2,00** | | | **5,00** | **5,00** | |
| Sodium Metabisulfite | | | **0,50** | **0,50** | | | |
| Troxerutin | **0,50** | **1,00** | **2,50** | **2,50** | **2,50** | **2,50** | **0,50** |
| Colorona® Bronze | | | | **5,00** | | **5,00** | **1,00** |
| Erythrulose | **2,00** | **2,00** | **2,00** | **2,00** | **2,00** | **2,00** | |
| Aqua (Water) | 10,00 | 10,00 | 10,00 | 10,00 | 10,00 | 10,00 | 10,00 |

| **Phase D** | | | | | | | |
|---|---|---|---|---|---|---|---|
| Parfum | 0,10 | 0,10 | 0,10 | 0,10 | 0,10 | 0,10 | 0,10 |
| | | | | | | | |
| **Complete** | **100,00** | **100,00** | **100,00** | **100,00** | **100,00** | **100,00** | **100,00** |

| **INCI** | **ST-08-50 %** | **ST-08-51 %** | **ST-08-52 %** | **ST-08-53 %** | **ST-08-54 %** |
|---|---|---|---|---|---|
| **Phase A** | | | | | |
| Glyeryl Stearate, Steareth-26, Ceteth-20, Stearyl Alcohol | 8,00 | 8,00 | 8,00 | 8,00 | 8,00 |
| Cetearyl Alcohol | 1,50 | 1,50 | 1,50 | 1,50 | 1,50 |
| Cetearyl Ethylhexanoate | 5,50 | 5,50 | 5,50 | 5,50 | 5,50 |
| Caprylic/Capric Tnglycende | 5,50 | 5,50 | 5,50 | 5,50 | 5,50 |
| Stearoxy Dimethicone | 1,00 | 1,00 | 1,00 | 1,00 | 1,00 |
| Dimethicone | 0,50 | 0,50 | 0,50 | 0,50 | 0,50 |
| Tocopheryl Acetate | 0,50 | 0,50 | 0,50 | 0,50 | 0,50 |
| Propylparaben | 0,05 | 0,05 | 0,05 | 0,05 | 0,05 |

| **Phase B** | | | | | |
|---|---|---|---|---|---|
| Ectoin | 0,30 | 0,30 | 0,30 | 0,30 | 0,30 |
| Propylene Glycol | 3,00 | 3,00 | 3,00 | 3,00 | 3,00 |
| Methylparaben | 0,15 | 0,15 | 0,15 | 0,15 | 0,15 |
| Aqua (Water) | 58,90 | 51,40 | 60,90 | 57,90 | 48,90 |

| **Phase C** | | | | | |
|---|---|---|---|---|---|
| Dihydroxyacetone | **2,00** | **5,00** | **1,00** | **2,00** | **5,00** |
| Dihydroxyacetone, Sodium, Metabisulfite, Magnesium stearate Sodium Metabisulfite Troxerutin | **1,00** | **2,50** | **0,50** | **1,00** | **2,50** |
| Colorona® Bronze | **2,00** | **5,00** | **1,00** | **2,00** | **5,00** |
| Erythrulose | | | **0,50** | **1,00** | **2,50** |
| Aqua (Water) | 10,00 | 10,00 | 10,00 | 10,00 | 10,00 |

| **Phase D** | | | | | |
|---|---|---|---|---|---|
| Parfum | 0,10 | 0,10 | 0,10 | 0,10 | 0,10 |
| | | | | | |
| **Complete** | **100,00** | **100,00** | **100,00** | **100,00** | **100,00** |

### Prozeß:

Man erhitzt Phasen A und B separat auf 80°C. Danach wird Phase B langsam unter Rühren zu Phase A gegeben und homogenisiert. Unter Rühren wird abgekühlt und die Phase C bei 40°C zugegeben und danach wird Phase D zugegeben.

## Patentansprüche

1. Verwendung von mindestens einem Flavonoid mit einem ungeladenen Flavangrundkörper zur Geruchsverbesserung und/oder Geruchsstabilisierung von Zubereitungen oder Vormischungen von Zubereitungen, enthaltend mindestens eine Selbstbräunungssubstanz, wobei das mindestens eine Flavonoid mit einem ungeladenen Flavangrundkörper die Selbstbräunungssubstanz stabilisiert und/oder wobei das mindestens eine Flavonoid mit einem ungeladenen Flavangrundkörper lagerabhängige Fehlgerüche der mindestens einen Selbstbräunungssubstanz reduziert oder vermeidet.

2. Verwendung nach Anspruch 1, **dadurch gekennzeichnet, dass** die Geruchsverbesserung bei kosmetischen, dermatologischen oder pharmazeutischen Zubereitungen oder Vormischungen für kosmetische, dermatologische oder pharmazeutische Zubereitungen stattfindet.

3. Verwendung nach Anspruch 1 oder 2, **dadurch gekennzeichnet, dass** die mindestens eine Selbstsbräunungssubstanz ausgewählt wird aus Dihydroxyaceton und/oder Erythrulose.

4. Verwendung nach einem oder mehreren der Ansprüche 1 bis 3, **dadurch gekennzeichnet, dass** das Flavonoid aus der Gruppe der Flavonole, Flavonol-O-glycoside , Flavonol- oder Flavonol-O-glycosid-enthaltende Extrakte ausgewählt wird.

5. Verwendung nach einem oder mehreren der Ansprüche 1 bis 4, **dadurch gekennzeichnet, dass** es sich bei dem mindestens einen Flavonoid um Quercetin, Rutin, Rutinsulfat, α-Glucosylrutin, Tilirosid, Troxerutin und/oder Isoquercetin handelt.

6. Verwendung nach einem oder mehreren der Ansprüche 1 bis 5, **dadurch gekennzeichnet, dass** es sich bei dem Flavonoid um ein Flavonoid handelt, bei dem eine oder mehrere phenolische Hydroxygruppen durch Veretherung oder Veresterung blockiert sind.

7. Verwendung nach Anspruch 6, **dadurch gekennzeichnet, dass** es sich um ein Hydroxyethyl-substituiertes Flavonoid, vorzugsweise Troxerutin, Troxequercetin, Troxeisoquercetin oder Troxeluteolin, handelt.

8. Verwendung nach Anspruch 6, **dadurch gekennzeichnet, dass** es sich um ein Flavonoid-sulfat oder Flavonoid-phosphat, vorzugsweise ein Rutinsulfat, handelt.

9. Verwendung nach einem oder mehreren der Ansprüche 1 bis 8, **dadurch gekennzeichnet, dass** das mindestens eine Flavonoid in einer Gesamtmenge von 0,01 bis 10 Gew.-%, bezogen auf die geruchsstabilisierte Zubereitung, eingesetzt wird.

10. Verwendung nach einem oder mehreren der Ansprüche 1 bis 9, **dadurch gekennzeichnet, dass** das mindestens eine Flavonoid in einer Gesamtmenge von 1 bis 95 Gew.-%, bezogen auf die geruchsstabilisierte Vormischung für eine Zubereitung, eingesetzt wird.

11. Verwendung nach einem oder mehreren der Ansprüche 1 bis 10, **dadurch gekennzeichnet, dass** das mindestens eine Flavonoid den Geruch von Zubereitungen, enthaltend mindestens eine Selbstbräuungssubstanz, auf der Haut stabilisiert und/oder verbessert.

12. Verwendung nach Anspruch 11, **dadurch gekennzeichnet, dass** die mindestens eine Selbstsbräunungssubstanz ausgewählt wird aus Dihydroxyaceton und/oder Erythrulose.

## Claims

1. Use of at least one flavonoid having an uncharged flavan skeleton for odour improvement and/or odour stabilisation of preparations or premixes of preparations comprising at least one self-tanning substance, where the at least one flavonoid having an uncharged flavan skeleton stabilises the self-tanning substance and/or where the at least one flavonoid having an uncharged flavan skeleton reduces or prevents malodours of the at least one self-tanning substance as a consequence of storage.

2. Use according to Claim 1, **characterised in that** the odour improvement takes place in cosmetic, dermatological or pharmaceutical preparations or premixes for cosmetic, dermatological or pharmaceutical preparations.

3. Use according to Claim 1 or 2, **characterised in that** the at least one self-tanning substance is selected from dihydroxyacetone and/or erythrulose.

4. Use according to one or more of Claims 1 to 3, **characterised in that** the flavonoid is selected from the group of the flavonols, flavonol O-glycosides, flavonol- or flavonol O-glycoside-containing extracts.

5. Use according to one or more of Claims 1 to 4, **characterised in that** the at least one flavonoid is quercetin, rutin, rutin sulfate, α-glucosylrutin, tiliroside, troxerutin and/or isoquercetin.

6. Use according to one or more of Claims 1 to 5, **characterised in that** the flavonoid is a flavonoid in which one or more phenolic hydroxyl groups have been blocked by etherification or esterification.

7. Use according to Claim 6, **characterised in that** the flavonoid is a hydroxyethyl-substituted flavonoid, preferably troxerutin, troxequercetin, troxeisoquercetin or troxeluteolin.

8. Use according to Claim 6, **characterised in that** the flavonoid is a flavonoid sulfate or flavonoid phosphate, preferably a rutin sulfate.

9. Use according to one or more of Claims 1 to 8, **characterised in that** the at least one flavonoid is employed in a total amount of 0.01 to 10% by weight, based on the odour-stabilised preparation.

10. Use according to one or more of Claims 1 to 9, **characterised in that** the at least one flavonoid is employed in a total amount of 1 to 95% by weight, based on the odour-stabilised premix for a preparation.

11. Use according to one or more of Claims 1 to 10, **characterised in that** the at least one flavonoid stabilises and/or improves the odour of preparations comprising at least one self-tanning substance on the skin.

12. Use according to Claim 11, **characterised in that** the at least one self-tanning substance is selected from dihydroxyacetone and/or erythrulose.

## Revendications

1. Utilisation d'au moins un flavonoïde ayant un squelette flavane non chargé pour l'amélioration de l'odeur et/ou la stabilisation de l'odeur de préparations ou de prémélanges de préparations comprenant au moins une substance auto-bronzante, où le au moins un flavonoïde ayant un squelette flavane non chargé stabilise la substance auto-bronzante et/ou où le au moins un flavonoïde ayant un squelette flavane non chargé réduit ou empêche les mauvaises odeurs de la au moins une substance auto-bronzante suite à un stockage.

2. Utilisation selon la revendication 1, **caractérisée en ce que** l'amélioration de l'odeur a lieu dans des préparations cosmétiques, dermatologiques ou pharmaceutiques ou des prémélanges pour des préparations cosmétiques, dermatologiques ou pharmaceutiques.

3. Utilisation selon la revendication 1 ou 2, **caractérisée en ce que** la au moins une substance auto-bronzante est choisie parmi la dihydroxyacétone et/ou l'érythrulose.

4. Utilisation selon l'une ou plusieurs parmi les revendications 1 à 3, **caractérisée en ce que** le flavonoïde est choisi dans le groupe constitué par les flavonols, les flavonol O-glycosides, les extraits contenant des flavonols ou des flavonol O-glycosides.

5. Utilisation selon l'une ou plusieurs parmi les revendications 1 à 4, **caractérisée en ce que** le au moins un flavonoïde est la quercétine, la rutine, le sulfate de rutine, l'α-glucosylrutine, le tiliroside, la troxérutine et/ou l'isoquercétine.

6. Utilisation selon l'une ou plusieurs parmi les revendications 1 à 5, **caractérisée en ce que** le flavonoïde est un flavonoïde dans lequel un ou plusieurs groupements hydroxyle phénoliques ont été bloqués par éthérification ou estérification.

7. Utilisation selon la revendication 6, **caractérisée en ce que** le flavonoïde est un flavonoïde substitué par hydroxyéthyle, préférablement la troxérutine, la troxéquercétine, la troxéisoquercétine ou la troxélutéoline.

8. Utilisation selon la revendication 6, **caractérisée en ce que** le flavonoïde est un sulfate de flavonoïde ou un phosphate de flavonoïde, préférablement un sulfate de rutine.

9. Utilisation selon l'une ou plusieurs parmi les revendications 1 à 8, **caractérisée en ce que** le au moins un flavonoïde est employé selon une quantité totale allant de 0,01 à 10% en poids sur la base de la préparation à odeur stabilisée.

10. Utilisation selon l'une ou plusieurs parmi les revendications 1 à 9, **caractérisée en ce que** le au moins un flavonoïde est employé selon une quantité totale allant de 1 à 95% en poids sur la base du prémélange à odeur stabilisée pour une préparation.

11. Utilisation selon l'une ou plusieurs parmi les revendications 1 à 10, **caractérisée en ce que** le au moins un flavonoïde stabilise et/ou améliore l'odeur de préparations comprenant au moins une substance auto-bronzante sur la peau.

12. Utilisation selon la revendication 11, **caractérisée en ce que** la au moins une substance auto-bronzante est choisie parmi la dihydroxyacétone et/ou l'érythrulose.
